# EUROPEAN PATENT SPECIFICATION

(11) **EP 1 781 274 B1**
(45) Date of publication and mention of the grant of the patent: **05.11.2008**
(21) Application number: 05782445.0
(22) Date of filing: 12.08.2005
(51) Int. Cl.: A61K 31/137, C07C 233/87, A61P 17/00

(54) **NOVEL BIAROMATIC COMPOUNDS WHICH ACTIVATE RECEPTORS OF PPAR TYPE AND THEIR USE IN COSMETIC OR PHARMACEUTICAL COMPOSITIONS**
NEUARTIGE BIAROMATISCHE PPAR-REZEPTOREN AKTIVIERENDE ZUSAMMENSETZUNGEN UND IHRE VERWENDUNG IN KOSMETISCHEN ODER PHARMAZEUTISCHEN ZUSAMMENSETZUNGEN
NOUVEAUX COMPOSES BIOAROMATIQUES ACTIVANT LES RECEPTEURS DE TYPE PPAR ET LEUR UTILISATION DANS DES COMPOSITIONS COSMETIQUES OU PHARMACEUTIQUES

(30) Priority: 17.08.2004 FR 0408932; 08.09.2004 US 607782 P
(43) Date of publication of application: 09.05.2007
(73) Proprietor: Galderma Research & Development, 06410 Biot (FR)
(72) Inventor: CLARY, Laurence, F-06480 La Colle sur Loup (FR); BOITEAU, Jean-Guy, F-34130 Saint-Aunes (FR); MILLOIS BARBUIS, Corinne, 83700 Saint Raphaël (FR)
(74) Representative: Allab, Myriam
(86) International application number: PCT/EP2005/009989
(87) International publication number: WO 2006/018325

(56) References cited:
- WO-A-01/16120
- WO-A-02/12210
- WO-A-03/055867

## Description

The invention relates, as novel and useful industrial products, to a novel class of biaromatic compounds which are activators of receptors of Peroxisome Proliferator-Activated Receptor type of subtype γ (PPARγ). It also relates to their process of preparation and to their use in pharmaceutical compositions intended for use in human or veterinary medicine or alternatively in cosmetic compositions.

The activity of receptors of PPAR type has formed the subject of many studies. Mention may be made, by way of indication, of the publication entitled "Differential Expression of Peroxisome Proliferator-Activated Receptor Subtypes During the Differentiation of Human Keratinocytes", Michel Rivier et al., J. Invest. Dermatol., 111, 1998, pp 1116-1121, in which a large number of bibliographic references relating to receptors of PPAR type are listed. Mention may also be made, by way of indication, of the report entitled "The PPARs: From Orphan Receptors to Drug Discovery", Timothy M. Willson, Peter J. Brown, Daniel D. Sternbach and Brad R. Henke, J. Med. Chem., 2000, Vol. 43, pp 527-550.

PPAR receptors activate transcription by binding to elements of DNA sequences, known as peroxisome proliferator response elements (PPRE), in the form of a heterodimer with retinoid X receptors (known as RXRs).

Three subtypes of human PPARs have been identified and described: PPARα, PPARγ and PPARδ (or NUC1).

PPARα is mainly expressed in the liver, while PPARδ is ubiquitous.

PPARγ is the most widely studied of the three subtypes. All the references suggest a critical role for PPARγs in the regulation of the differentiation of adipocytes, where it is strongly expressed. It also plays a key role in systemic lipid homeostasis.

It has been disclosed in particular in Patent Application WO 96/33724 that compounds which are selective for PPARγs, such as a prostaglandin J₂ or D₂, are potential active principles in the treatment of obesity and diabetes.

Furthermore, the Applicant Company has already disclosed, in Patent Application WO 02/12210 and WO 03/055867, the use of biaromatic compounds which are activators of receptors of PPARγ type in the preparation of a pharmaceutical composition, the composition being intended for the treatment of skin disorders related to an anomaly in the differentiation of the epidermal cells.

It does not thereby remain any less the case that it is still necessary to search for novel compounds exhibiting a good activity and advantageous pharmaceutical properties.

The Applicant Company has now identified a novel family of derivatives exhibiting the advantage of being 10 to 100 times more active than the compounds identified in the preceding Patent Applications WO 02/12210 and WO 03/055867 with regard to PPARγ receptors. Furthermore, in addition to their better activity, some of the compounds according to the present invention are obtained in the solid form. The synthesis thereof and the purification thereof are easier.

Finally, the use of solid compounds makes it possible to avoid the disadvantages exhibited by oils in the context of the pharmaceutical development due to the residual solvents which may be present therein.

Thus, the present invention relates to compounds corresponding to the following general formula (I): 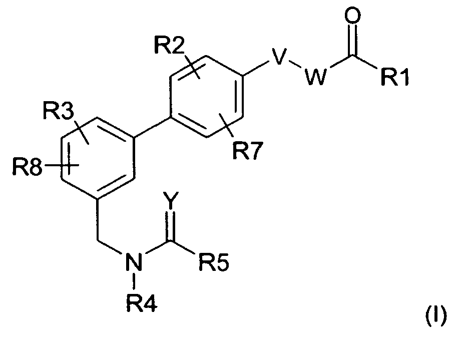 in which:
- **R1** represents a hydroxyl radical, an -OR6 radical or a hydroxylamine radical; R6 being defined below,
- **R2** and **R3,** which are identical or different, represent a hydrogen atom, a halogen atom, an alkyl radical comprising 1 to 12 carbon atoms, a hydroxyl radical, an alkoxy radical, a polyether radical, an aralkyl radical, an aryl radical or an amino radical which can be substituted by one or two identical or different radicals chosen from an alkyl radical comprising 1 to 12 carbon atoms or an aralkyl radical;
- **R4** represents a hydrogen atom or a lower alkyl radical having from 1 to 4 carbon atoms;
- **R5** represents an alkyl radical having from 1 to 12 carbon atoms, an aryl radical, an aralkyl radical, a heteroaryl radical, a heterocyclic radical or a 9-fluorenylmethyl radical;
- **R6** represents an alkyl, aryl or aralkyl radical;
- **R7** and **R8,** which are identical or different, represent a hydrogen atom, a halogen atom, an alkyl radical comprising 1 to 12 carbon atoms, a hydroxyl radical, an alkoxy radical, a polyether radical, an aralkyl radical, an aryl radical or an amino radical which can be substituted by one or two identical or different radicals chosen from an alkyl radical comprising 1 to 12 carbon atoms or an aralkyl radical;
- **Y** represents an oxygen or sulphur atom;
- **V-W** represents a CH₂-CH₂ or CH = CH sequence,
and the salts of the compounds of formula (I).

In particular, when the compounds according to the invention are provided in the form of salts, they are salts of an alkali metal, in particular a sodium or potassium salt, or of an alkaline earth metal or salts of organic amines, more particularly of amino acids, such as arginine or lysine.

When the compounds according to the invention have an amine functional group and are provided in the form of salts of this amine, they are salts of an inorganic acid, such as, for example, hydrochloric acid, sulphuric acid or hydrobromic acid, or salts of an organic acid, such as, for example, acetic acid, triflic acid, tartaric acid, oxalic acid, citric acid or nitric acid.

According to the present invention, the term "hydroxyl radical" is understood to mean the -OH radical.

According to the present invention, the term "alkyl radical comprising 1 to 12 carbon atoms" is understood to mean a linear, branched or cyclic and saturated or unsaturated carbon chain which can be interrupted by a heteroatom and which can be substituted by one or more radicals chosen from a halogen atom, a hydroxyl radical, an alkoxy radical or a heterocyclic radical and the alkyl radicals are preferably the methyl, ethyl, propyl, isopropyl, butyl, tert-butyl, isoamyl, amyl, hexyl, heptyl, octyl, decyl, cyclohexyl, methylcyclohexyl, methylcyclobutyl, methylcyclopentyl or methylcyclohexyl radicals.

The term "lower alkyl radical comprising 1 to 4 carbon atoms" will preferably mean a methyl, ethyl, propyl, methylcyclopropyl, isopropyl, tert-butyl or n-butyl radical.

The term "aryl radical" is understood to mean a phenyl, biphenyl, cinnamyl or naphthyl radical which can be substituted by a halogen atom, a CF₃ radical, an alkyl radical, an alkoxy radical, a nitro functional group, a polyether radical, an aryl radical, a benzoyl radical, an alkyl ester group, a carboxylic acid, a hydroxyl radical optionally protected by an acetyl or benzoyl group or an amino radical optionally protected by an acetyl or benzoyl group or optionally substituted by at least one alkyl having from 1 to 12 carbon atoms, an aralkoxy radical, a phenoxy radical or an amide radical H₂NCO.

The term "aralkyl radical" is understood to mean an alkyl radical comprising 1 to 12 carbon atoms which is substituted by an aryl radical or by a heteroaryl radical.

The term "halogen atom" is understood to mean a fluorine, chlorine, bromine or iodine atom.

The term "alkoxy radical" is understood to mean an oxygen atom substituted by an alkyl radical comprising 1 to 12 carbon atoms and the alkoxy radicals are preferably the methoxy, ethoxy, isopropyloxy, n-propyloxy, tert-butoxy, n-butoxy, n-pentyloxy, n-hexyloxy, cyclopropylmethoxy, cyclobutylmethoxy, cyclopentylmethoxy or cyclohexylmethoxy radicals.

The term "aralkoxy radical" is understood to mean an oxygen atom substituted by an aralkyl radical.

The term "polyether radical" is understood to mean a radical having from 1 to 7 carbon atoms which is interrupted by at least one oxygen atom and preferably the radicals such as methoxyethoxy, ethoxyethoxy or methoxyethoxyethoxy radicals.

The term "heteroaryl radical" is understood to mean an aryl radical which is interrupted by one or more heteroatoms, such as the pyridyl, furyl, thienyl, isoxazolyl, oxadiazolyl, oxazolyl, isothiazolyl, quinozalinyl, benzothiadiazolyl, benzimidazolyl, indolyl or benzofuranyl radical, and which is optionally substituted by at least one halogen, one alkyl radical having from 1 to 12 carbon atoms, one alkoxy radical, one aryl radical, one nitro functional group, one polyether radical, one heteroaryl radical, one benzoyl radical, one alkyl ester group, one carboxylic acid, one hydroxyl optionally protected by an acetyl or benzoyl group or one amino radical optionally protected by an acetyl or benzoyl group or optionally substituted by at least one alkyl having from 1 to 12 carbon atoms.

The term "heterocyclic radical" is understood to mean preferably a morpholino, pyrrolidino, piperidino, piperazino, 2-oxopiperidin-1-yl and 2-oxopyrrolidin-1-yl radical which are optionally substituted by at least one alkyl radical having from 1 to 12 carbon atoms, one alkoxy radical, one aryl radical, one nitro functional group, one polyether radical, one heteroaryl radical, one benzoyl radical, one alkyl ester group, one carboxylic acid, one hydroxyl optionally protected by an acetyl or benzoyl group or one amino radical optionally protected by an acetyl or benzoyl group or optionally substituted by at least one alkyl having from 1 to 12 carbon atoms.

The term "hydroxylamine radical" is understood to mean the - NHOH sequence.

The term "alkyl ester radical" is understood to mean a carboxylate functional group substituted by an alkyl radical having 1 to 6 carbon atoms.

Mention may in particular be made, among the compounds of formula (I) above coming within the scope of the present invention, of the following compounds (alone or as a mixture):
1- 3-{3'-[((Methyl)(octanoyl)amino)methyl]biphenyl-4-yl}acrylic acid
2- 3-{3'-[((Methyl)(octanoyl)amino)methyl]biphenyl-4-yl}propanoic acid
3- 3-{3-Fluoro-3'-[((methyl)(octanoyl)amino)methyl]biphenyl-4-yl}acrylic acid
4- 3-{3-Fluoro-3'-[((methyl)(octanoyl)amino)methyl]biphenyl-4-yl}propanoic acid
5- 3-[3'-((Octanoylamino)methyl)biphenyl-4-yl]acrylic acid
6- 3-[3'-((Octanoylamino)methyl)biphenyl-4-yl]propanoic acid
7- 3-{3-Hydroxy-3'-[((methyl)(octanoyl)amino)methyl]biphenyl-4-yl}propanoic acid
8- 3-{2-Butoxy-3'-[((methyl)(octanoyl)amino)methyl]biphenyl-4-yl}propanoic acid
9- 3-{2-Butoxy-3'-[((hexanoyl)(methyl)amino)methyl]biphenyl-4-yl}propanoic acid
10- 3-{2-Butoxy-3'-[((methyl)(pentanoyl)amino)methyl]biphenyl-4-yl}propanoic acid
11- 3-{2-Butoxy-3'-[((heptanoyl)(methyl)amino)methyl]biphenyl-4-yl}propanoic acid
12- 3-(2-Butoxy-3'-{[(4-ethoxybenzoyl)(methyl)amino]-methyl}biphenyl-4-yl)propanoic acid
13- 3-(2-Butoxy-3'-{[(4-butoxybenzoyl)(methyl)amino]-methyl}biphenyl-4-yl)propanoic acid
14- 3-{3'-[((Benzoyl)(methyl)amino)methyl]-2-butoxy-biphenyl-4-yl}propanoic acid
15- 3-(2-Butoxy-3'-{[(4-methoxybenzoyl)(methyl)amino]-methyl}biphenyl-4-yl)propanoic acid
16- 3-(2-Butoxy-3'-{[(3-methoxybenzoyl)(methyl)amino]-methyl}biphenyl-4-yl)propanoic acid
17- (E)-3-{2-Fluoro-3'-[((methyl)(octanoyl)amino)methylJbiphenyt-4-yl}acrylic acid
18- 3-{2-Fluoro-3'-[((methyl)(octanoyl)amino)methyl]biphenyl-4-yl}propanoic acid
19- 3-{2-(2-Methoxyethoxy)-3'-[((methyl)(octanoyl)amino)methyl]biphenyl-4-yl}propanoic acid
20- 3-{2-(3-Methylbutoxy)-3'-[((methyl)(octanoyl)amino)methyl]biphenyl-4-yl}propanoic acid
21- 3-{2-(3-Chloropropoxy)-3'-[((methyl)(octanoyl)amino)methyl]biphenyl-4-yl}propanoic acid
22- 3-{2-Methoxy-3'-[((methyl)(octanoyl)amino)methyl]biphenyl-4-yl}propanoic acid
23- 3-{2-Methyl-3'-[((methyl)(octanoyl)amino)methyl]biphenyl-4-yl}propanoic acid
24- 3-(3'-{[Methyl(4-phenylbutyryl)amino]methyl}-biphenyl-4-yl)propanoic acid
25- (E)-3-{2'-Methyl-5'-[((methyl)(octanoyl)amino)methyl]biphenyl-4-yl}acrylic acid
26- 3-{2'-Methyl-5'-[((methyl)(octanoyl)amino)methyl]biphenyl-4-yl}propanoic acid
27- (E)3-{2-Benzyloxy-3'-[((methyl)(octanoyl)amino)methyl]biphenyl-4-yl}acrylic acid
28- 3-{2-Benzyloxy-3'-[((methyl)(octanoyl)amino)methyl]biphenyl-4-yl}propanoic acid
29- 3-{3'-[((Methyl)(octanoyl)amino)methyl]-2-propoxy-biphenyl-4-yl}propanoic acid
30- (E)-3-{3,5-Difluoro-3'-[((methyl)(octanoyl)amino)methyl]biphenyl-4-yl}acrylic acid
31- 3-{3,5-Difluoro-3'-[((methyl)(octanoyl)amino)methyl]biphenyl-4-yl}propanoic acid
32- 3-(3'-{[(4-Butoxybenzoyl)(methyl)amino]methyl}-biphenyl-4-yl)propanoic acid
33- 3-(3'-{[(4-Butoxybenzoyl)(ethyl)amino]methyl}-biphenyl-4-yl)propanoic acid
34- 3-{2-Cyclopropylmethoxy-3'-[((methyl)(octanoyl)amino)methyl]biphenyl-4-yl}propanoic acid
35- 3-{2-Ethoxy-3'-[((methyl)(octanoyl)amino)methyl]biphenyl-4-yl}propanoic acid
36- 3-[3'-[((Methyl)(octanoyl)amino)methyl]-2-(3,3,3-trifluoropropoxy)biphenyl-4-yl]propanoic acid
37- 3-[3'-[((Methyl)(octanoyl)amino)methyl]-2-(4,4,4-trifluorobutoxy)biphenyl-4-yl]propanoic acid
38- 3-{2-(3-Hydroxypropoxy)-3'-[((methyl)(octanoyl)amino)methyl]biphenyl-4-yl}propanoic acid
39- 3-{2-(4-Hydroxybutoxy)-3'-[((methyl)(octanoyl)amino)methyl]biphenyl-4-yl}propanoic acid
40- 3-{2-(3-Fluorobenzyloxy)-3'-[((methyl)(octanoyl)amino)methyl]biphenyl-4-yl}propanoic acid
41- 3-{2-(4-Fluorobenzyloxy)-3'-[((methyl)(octanoyl)amino)methyl]biphenyl-4-yl}propanoic acid
42- 3-{3'-[((Methyl)(octanoyl)amino)methyl]-2-(pentyloxy)biphenyl-4-yl}propanoic acid
43- 3-{3'-[((Hexanoyl)(methyl)amino)methyl]-2-(pentyloxy)biphenyl-4-yl}propanoic acid
44- 3-{2-(2-Ethoxyethoxy)-3'-[((hexanoyl)(methyl)amino)methyl]biphenyl-4-yl}propanoic acid
45- 3-{2-(2-(Diethylamino)ethoxy)-3'-[((methyl)(octanoyl)amino)methyl]biphenyl-4-yl}propanoic acid
46- 3-[3'-[((Methyl)(octanoyl)amino)methyl]-2-(2-(morpholin-4-yl)ethoxy)biphenyl-4-yl]propanoic acid
47- 3-{2-Amino-3'-[((hexanoyl)(methyl)amino)methyl]biphenyl-4-yl}propanoic acid
48- 3-{2-Butylamino-3'-[((hexanoyl)(methyl)amino)methyl]biphenyl-4-yl}propanoic acid
49- 3-{2-Benzylamino-3'-[((hexanoyl)(methyl)amino)methyl]biphenyl-4-yl}propanoic acid
50- 3-{2-Diethylamino-3'-[((hexanoyl)(methyl)amino)methyl]biphenyl-4-yl}propanoic acid
51- 3-{3'-[((Hexanoyl)(methyl)amino)methyl]-2-(propylamino)biphenyl-4-yl}propanoic acid
52- 3-{2-(4-Fluorobenzylamino)-3'-[((hexanoyl)(methyl)amino)methyl]biphenyl-4-yl}propanoic acid
53- 3-{2-Butylamino-3'-[((methyl)(octanoyl)amino)methyl]biphenyl-4-yl}propanoic acid
54- 3-{2-Benzylamino-3'-[((methyl)(octanoyl)amino)methyl]biphenyl-4-yl}propanoic acid
55- 3-{2-Diethylamino-3'-[((methyl)(octanoyl)amino)methyl]biphenyl-4-yl}propanoic acid
56- 3-{3'-[((Methyl)(octanoyl)amino)methyl]-2-(propylamino)biphenyl-4-yl}propanoic acid
57- 3-{2-(4-Fluorobenzylamino)-3'-[((methyl)(octanoyl)amino)methyl]biphenyl-4-yl}propanoic acid
58- 3-{2-Cyclohexylmethoxy-3'-[((methyl)(octanoyl)amino)methyl]biphenyl-4-yl}propanoic acid
59- 3-{2-Cyclopentylmethoxy-3'-[((methyl)(octanoyl)amino)methyl]biphenyl-4-yl}propanoic acid
60- N-[2'-(2-Cyclobutylethoxy)-4'-(2-(hydroxycarbamoyl)ethyl)biphenyl-3-ylmethyl](methyl)octanamide
61- 3-[3'-[((Methyl)(octanoyl)amino)methyl]-2-(3-(trifluoromethyl)benzyloxy)biphenyl-4-yl]propanoic acid
62- 3-[3'-[((Hexanoyl)(methyl)amino)methyl]-2-(4-(trifluoromethyl)benzyloxy)biphenyl-4-yl]propanoic acid
63- 3-{2-(3-Carbamoylbenzyloxy)-3'-[((methyl)(octanoyl)amino)methyl]biphenyl-4-yl}propanoic acid
64- 3-[3'-[((Methyl)(octanoyl)amino)methyl]-2-(2-(piperazin-1-yl)ethoxy)biphenyl-4-yl]propanoic acid
65- 3-[3'-[((Methyl)(octanoyl)amino)methyl]-2-(2-(pyrrolidin-1-yl)ethoxy)biphenyl-4-yl]propanoic acid
66- 3-[2-(3-Methoxybenzyloxy)-3'-({[3-(3-methoxyphenyl)propionyl](methyl)amino}methyl)biphenyl-4-yl]propanoic acid
67- 3-[2-(4-(tert-Butyl)benzyloxy)-3'-({methyl[3-(3-phenoxyphenyl)propionyl]amino}methyl)biphenyl-4-yl]propanoic acid
68- 3-(2-(3,5-Dimethoxybenzyloxy)-3'-{[methyl(3-phenoxybenzoyl)amino]methyl}biphenyl-4-yl)propanoic acid
69- 3-[3'-{[Methyl(4-phenoxybenzoyl)amino]methyl}-2-(3-(trifluoromethyl)benzyloxy)biphenyl-4-yl]propanoic acid
70- 3-[2-(3-Isopropoxybenzyloxy)-3'-({[3-(4-methoxyphenyl)propionyl](methyl)amino}methyl)biphenyl-4-yl]propanoic acid
71- 3-[2'-(3-Methoxybenzyloxy)-5'-({[3-(3-methoxyphenyl)propionyl](methyl)amino}methyl)biphenyl-4-yl]propanoic acid
72- 3-{2'-Cyclohexylmethoxy-5'-[((hexanoyl)(methyl)amino)methyl]biphenyl-4-yl}propanoic acid
73- 3-{4'-Ethoxy-3'-[((hexanoyl)(methyl)amino)methyl]-2-propoxybiphenyl-4-yl}propanoic acid
74- 3-{3'-[((Hexanoyl)(methyl)amino)methyl]-3,5-dimethoxybiphenyl-4-yl}propanoic acid
75- 3-[3,5-Diethoxy-3'-({[3-(3-methoxyphenyl)-propionyl](methyl)amino}methyl)biphenyl-4-yl]propanoic acid
76- 3-[3'-({[3-(4-Methoxyphenyl)propionyl](methyl)amino}methyl)-3-propoxybiphenyl-4-yl]propanoic acid
77- 3-{3-Cyclopropylmethoxy-3'-[((hexanoyl)(methyl)amino)methyl]biphenyl-4-yl}propanoic acid
78- 3-{3-Ethoxy-4'-fluoro-3'-[((hexanoyl)(methyl)amino)methyl]biphenyl-4-yl}propanoic acid
79- 3-[3'-[((Hexanoyl)(methyl)amino)methyl]-3-(4,4,4-trifluorobutoxy)biphenyl-4-yl]propanoic acid
80- 3-{3-Benzyloxy-3'-[((hexanoyl)(methyl)amino)methyl]biphenyl-4-yl}propanoic acid
81- 3-[3'-({[3-(4-Methoxyphenyl)propionyl](methyl)amino}methyl)-3-(3-(trifluoromethyl)benzyloxy)biphenyl-4-yl]propanoic acid
82- 3-[3'-({[3-(3-Methoxyphenyl)propionyl](methyl)amino}methyl)-3,5-dipropylbiphenyl-4-yl]propanoic acid
83- 3-{3-(2,2-Dimethylpropyl)-3'-[((hexanoyl)(methyl)amino)methyl]biphenyl-4-yl}propanoic acid
84- 3-{3'-[((Hexanoyl)(methyl)amino)methyl]-3,5-dimethylbiphenyl-4-yl}propanoic acid
85- 3-{3-[((Hexanoyl)(methyl)amino)methyl]-4"-methoxy-1,1';3',1"-terphenyl-4'-yl}propanoic acid
86- 3-{3-[((Hexanoyl)(methyl)amino)methyl]-3"-methoxy-1,1';2',1"-terphenyl-4'-yl}propanoic acid
87- 3-[3-({[3-(3-Methoxyphenyl)propionyl](methyl)amino}methyl)-3"-trifluoromethyl-1,1';2',1 "-terphenyl-4'-yl]propanoic acid
88- 3-{3'-[((Hexanoyl)(methyl)amino)methyl]-2-[2-(3-isopropoxyphenyl)ethyl]biphenyl-4-yl}propanoic acid
89- 3-{3'-[((Hexanoyl)(methyl)amino)methyl]-2-[(pyridin-3-ylmethyl)amino]biphenyl-4-yl}propanoic acid
90- 3-[3'-[((Hexanoyl)(methyl)amino)methyl]-3-(2-methoxyethylamino)biphenyl-4-yl]propanoic acid
91- Methyl 3-{3,5-diethyl-3'-[((hexanoyl)(methyl)amino)methyl]biphenyl-4-yl}propanoate
92- Methyl 3-[3'-{[methyl(3-phenoxybenzoyl)amino]-methyl}-2-(3-(trifluoromethyl)benzyloxy)biphenyl-4-yl]propanoate
93- Methyl 3-[3'-{[(3-(biphenyl-4-yl)propionyl)(methyl)amino]methyl}-2-(3-methoxybenzyloxy)biphenyl-4-yl]propanoate
94- Ethyl 3-[3'-({[3-(3-methoxyphenyl)propionyl](methyl)amino}methyl)-2-(4,4,4-trifluorobutoxy)biphenyl-4-yl]propanoate
95- N-[4'-(2-(Hydroxycarbamoyl)ethyl)-4"--methoxy-1,1';3',1"-terphenyl-3-ylmethyl](methyl)hexanamide.

According to the present invention, the compounds of formula (I) which are more particularly preferred are those which exhibit at least one of the following characteristics:
- **R1** represents a hydroxyl radical;
- **R2** and **R7** represent an alkoxy or aryloxy radical, an alkylamino radical or a polyether radical;
- **R3** and **R8** represent a hydrogen atom;
- **R4** represents a lower alkyl radical comprising 1 to 4 carbon atoms;
- **R5** represents an alkyl radical comprising 3 to 8 carbon atoms or an aryl radical;
- **Y** is an oxygen atom;
- the **V-W** bond is CH₂-CH₂ or CH=CH.

In particular, preference will be given to the compounds of general formula (I) exhibiting all of the following characteristics:
- **R1** represents a hydroxyl radical;
- **R2** and **R7** represent an alkoxy or aryloxy radical, an alkylamino radical or a polyether radical;
- **R3** and **R8** represent a hydrogen atom;
- **R4** represents a lower alkyl radical comprising 1 to 4 carbon atoms;
- **R5** represents an alkyl radical comprising 3 to 8 carbon atoms or an aryl radical;
- **Y** is an oxygen atom;
- the **V-W** bond is -CH₂-CH₂- or CH=CH.

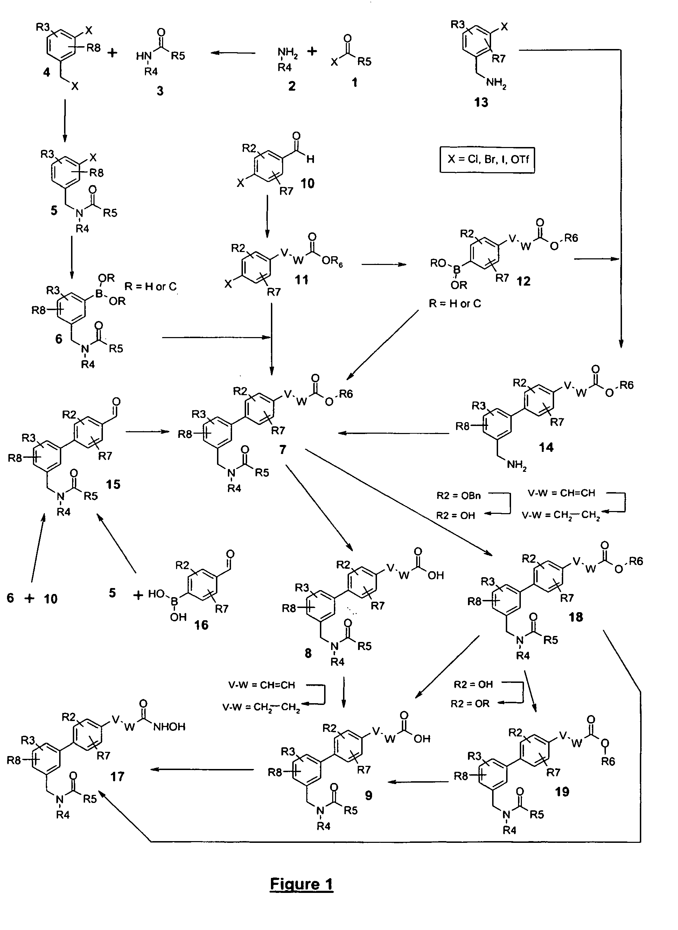

Another subject-matter of the present invention is the processes for the preparation of the compounds of formula (I), in particular according to the reaction schemes given in **Figure 1****.**

### Route 1-2-3-4-5-6-7-8-9

The amides **3** can be obtained by treating the corresponding amines **2** with acyl halides **1** possessing the desired chain. For example, octanoyl chloride can react with methylamine to provide the corresponding amide.

The amides **3** can react with benzyl halides **4** in the presence of a base, such as NaH, for example, to give the compounds **5.**

The boronic ester **6** can be obtained by treating compound **5** with bis(pinacolato)diboron, for example in the presence of a palladium-based catalyst, such as [1,1'-bis(diphenylphosphino)ferrocene]dichloropalladium(II). Its corresponding boronic acid (R = H) can be obtained by using conventional conditions, such as, for example, the use of tert-butyllithium, followed by addition to trimethyl borate.

The aryl halides or triflates **11** can be obtained commercially or else can be synthesized by a Wittig reaction by treating the suitably substituted aldehydes 10 with methyl (triphenylphosphoranylidene)acetate, for example.

A palladium coupling of Suzuki type between the boronate **6** and the appropriate reactant **11** (aryl bromide, iodide, chloride or triflate) makes it possible to obtain the compound exhibiting the aryl-aryl sequence **7.**

The acid functional group of the compound **8** can be obtained from 7 by saponification, if R6 is an alkyl chain, with a base, such as sodium hydroxide, or by hydrogenolysis, if R6 is a benzyl.

The compound **9** can be obtained by hydrogenation, if **8** has V-W = CH = CH, under conventional hydrogenation conditions, such as, for example, hydrogen catalysed by palladium-on-charcoal.

The compound **17** can be obtained from the acid **9** by treatment with oxalyl chloride, for example, followed by reaction with hydroxylamine.

The compound **17** can be obtained from the ester **18** by treatment with hydroxylamine.

The compound **7** can be subjected to a deprotection/protection sequence if R5 = O-(t-Bu). In this case, treatment with trifluoroacetic acid, for example, followed by reaction with an acyl chloride, can result in the compounds where R5 = alkyl.

In the case where R2 = OBn, a hydrogenation reaction, in the presence of palladium-on-charcoal, for example, and under a hydrogen atmosphere, can be carried out on the compound **7** to provide the compounds **18** in which R2 = OH.

The compounds **19** can be obtained by alkylation of the phenol under conventional conditions, such as, for example, in the presence of an alkyl halide and of potassium carbonate.

The acid functional group of the compound **9** can be obtained from **18** or **19** by saponification, if R6 is an alkyl chain, with a base, such as sodium hydroxide.

### Route 10-11-12-16-14-7-8-9

The compounds **11** can be obtained from the corresponding aldehydes **10** by a Wittig reaction with methyl (triphenylphosphoranylidene)acetate.

The boronic ester **12** can be obtained by treating the compound **11** with bis(pinacolato)diboron, for example in the presence of a palladium-based catalyst, such as [1,1'-bis(diphenylphosphino)ferrocene]dichloropalladium(II).

A palladium coupling of Suzuki type between the boronate **12** and the appropriate reactant **13** (bromide, iodide, chloride) makes it possible to obtain the compound exhibiting the-aryl-aryl sequence **14.**

The compound **14** can be subjected to a conventional acylation reaction with an acyl halide in order to provide the compound **7** in which R4 = H.

A palladium coupling of Suzuki type between the boronate **12** and the appropriate reactant **5** (bromide, iodide, chloride) makes it possible to obtain the compounds exhibiting the aryl-aryl sequence **7.**

### Route 6-10-15-7-8-9

A palladium coupling of Suzuki type between the boronate **6** (or the corresponding boronic acid) and the appropriate reactant **10** (aryl bromide, iodide, chloride or triflate) makes it possible to obtain the compound exhibiting the aryl-aryl sequence **15.**

The compound **7** can be obtained by a Wittig reaction by treating the corresponding compound **15** with methyl (triphenylphosphoranylidene)acetate, for example.

### Route 5-16-15-7-8-9

A palladium coupling of Suzuki type between the boronic acid **16** and the compound **5** (aryl bromide, iodide, chloride or triflate) makes it possible to obtain the compound exhibiting the aryl-aryl sequence **15.**

The compounds according to the invention exhibit modulatory properties with regard to receptors of PPAR type. This activity on PPARα, δ and γ receptors is measured in a transactivation test and quantified by the dissociation constant Kdapp (apparent), as described in Example 17.

The preferred compounds of the present invention exhibit a dissociation constant of less than or equal to 5000 nM and advantageously of less than or equal to 1000 nM.

Preferably, the compounds are modulators of specific receptors of PPARγ type, that is to say that they exhibit a ratio of the Kdapp for the PPARα or PPARδ receptors to the Kdapp for the PPARγ receptors of greater than or equal to 10. Preferably, this PPARα/PPARγ or PPARδ/PPARγ ratio is greater than or equal to 50 and more advantageously greater than or equal to 100.

Another subject-matter of the present invention is the compounds of formula (I) as described above as medicament.

The compounds according to the invention are particularly highly suitable in the following fields of treatment:
1) for treating dermatological conditions linked to a disorder of keratinization involving differentiation and proliferation, in particular for treating acne vulgaris, comedonic or polymorphic acne, acne rosacea, nodulocystic acne, acne conglobata, senile acne or secondary acnes, such as solar, drug or occupational acne,
2) for treating other types of disorders of keratinization, in particular ichthyoses, ichthyosiform conditions, Darier's disease, palmoplantar keratoderma, leucoplakia and leucoplakiform conditions, or cutaneous or mucosal (oral) lichen,
3) for treating other dermatological conditions with an inflammatory immunoallergic component, with or without cell proliferation disorder, and, in particular, all forms of psoriasis, whether cutaneous, mucosal or ungual, and even psoriatic rheumatism, or alternatively cutaneous atopy, such as eczema, or respiratory atopy or alternatively gingival hypertrophy,
4) for treating all dermal or epidermal proliferations, whether they are benign or malignant and whether they are or are not of viral origin, such as common warts, flat warts and epidermodysplasia verruciformis, florid or oral papillomatoses, T lymphoma, and the proliferations which can be induced by ultraviolet radiation, in particular in the case of basal cell and prickle cell epithelioma, and also all precancerous skin lesions, such as keratoacanthomas,
5) for treating other dermatological disorders, such as immune dermatoses, such as lupus erythematosus, immune bullous diseases and collagen diseases, such as scleroderma,
6) in the treatment of dermatological or general conditions with an immunological component,
7) in the treatment of skin disorders due to exposure to UV radiation, and also for repairing or combating skin ageing, whether photoinduced or chronologic, or for reducing actinic keratoses and pigmentations, or any pathology associated with chronologic or actinic ageing, such as xerosis,
8) for combating disorders of the sebaceous function, such as hyperseborrhoea of acne or simple seborrhoea,
9) for preventing or treating disorders of cicatrization or for preventing or repairing stretch marks,
10) in the treatment of disorders of pigmentation, such as hyperpigmentation, melasma, hypopigmentation or vitiligo,
11) in the treatment of conditions of the metabolism of lipids, such as obesity, hyperlipidaemia or non-insulin-dependent diabetes,
12) in the treatment of inflammatory conditions, such as arthritis,
13) in the treatment or prevention of cancerous or precancerous conditions,
14) in the prevention or treatment of alopecia of various origins, in particular alopecia due to chemotherapy or to radiation,
15) in the treatment of disorders of the immune system, such as asthma, type I diabetes mellitus, multiple sclerosis or other selective dysfunctions of the immune system,
16) in the treatment of conditions of the cardiovascular system, such as arteriosclerosis or hypertension.

Another subject-matter of the present invention is a pharmaceutical or cosmetic composition comprising, in a physiologically acceptable medium, at least one compound of formula (I) as defined above.

Another subject-matter of the present invention is the use of the compounds of formula (I) in the manufacture of a composition intended for the treatment of the abovementioned conditions, in particular for regulating and/or restoring the metabolism of skin lipids.

The composition according to the invention can be administered orally, enterally, parenterally, topically or ocularly. Preferably, the pharmaceutical composition is packaged in a form suitable for topical application.

Orally, the composition, more particularly the pharmaceutical composition, can be provided in the form of tablets, including sugar-coated tablets, hard gelatin capsules, syrups, suspensions, solutions, powders, granules, emulsions or lipid or polymeric microspheres or nanospheres or vesicles which make possible controlled release. Parenterally, the composition can be provided in the form of solutions or suspensions for infusion or for injection.

The compounds according to the invention are generally administered at a daily dose of approximately 0.001 mg/kg to 100 mg/kg of body weight, taken 1 to 3 times.

The compounds are used systemically at a concentration generally of between 0.001% and 10% by weight, preferably between 0.01% and 1% by weight, with respect to the weight of the composition.

Topically, the pharmaceutical composition according to the invention is more particularly intended for the treatment of the skin and mucous membranes and can be provided in the form of salves, creams, milks, ointments, powders, impregnated pads, solutions, gels, sprays, lotions or suspensions. It can also be provided in the form of lipid or polymeric microspheres or nanospheres or vesicles or of polymeric patches and of hydrogels which make possible controlled release. This topical composition can be provided in the anhydrous form, in the aqueous form or in the form of an emulsion.

The compounds are used topically at a concentration generally of between 0.001% and 10% by weight, preferably between 0.01% and 1% by weight, with respect to the total weight of the composition.

The compounds of formula (I) according to the invention also have an application in the cosmetics field, in particular in body and hair hygiene and more particularly for regulating and/or restoring the metabolism of skin lipids. In comparison with the products known previously, these compounds of formula (I) have the advantage of additionally exhibiting other advantageous properties, in particular antiinflammatory or soothing properties, which makes them less irritating and therefore better tolerated compounds.

Another subject-matter of the invention is thus the cosmetic use of a composition comprising, in a physiologically acceptable vehicle, at least one of the compounds of formula (I) for body or hair hygiene.

The cosmetic composition according to the invention, comprising, in a cosmetically acceptable vehicle, at least one compound of formula (I) or one of its optical or geometrical isomers or one of its salts, can be provided in particular in the form of a cream, a milk, a lotion, a gel, lipid or polymeric microspheres or nanospheres or vesicles, a soap or a shampoo.

The concentration of compound of formula (I) in the cosmetic composition is between 0.001 % and 3% by weight, with respect to the total weight of the composition.

The compositions as described above can, of course, additionally comprise inert or even pharmacodynamically active additives or combinations of these additives and in particular: wetting agents; depigmenting agents, such as hydroquinone, azelaic acid, caffeic acid or kojic acid; emollients; moisturizing agents, such as glycerol, polyethylene glycol (PEG) 400, thiamorpholinone and its derivatives, or urea; antiseborrhoeic or antiacne agents, such as S-carboxymethylcysteine, S-benzylcysteamine, their salts or their derivatives, or benzoyl peroxide; antifungal agents, such as ketoconazole or 4,5-polymethylene-3-isothiazolidones; antibacterials; carotenoids and in particular β-carotene; antipsoriatic agents, such as anthralin and its derivatives; eicosa-5,8,11,14-tetraynoic and eicosa-5,8,11-triynoic acids, their esters and amides; and, finally, retinoids. The compounds of formula (I) can also be combined with vitamins D or their derivatives, with corticosteroids, with agents for combating free radicals, with α-hydroxy or α-keto acids or their derivatives, or with ion-channel blockers.

These compositions can also comprise flavour enhancers, preservatives, such as esters of para-hydroxybenzoic acid, stabilizing agents, moisture-regulating agents, pH-regulating agents, agents for modifying osmotic pressure, emulsifying agents, UV-A and UV-B screening agents, or antioxidants, such as α-tocopherol, butylated hydroxyanisole or butylated hydroxytoluene.

Of course, a person skilled in the art will take care to choose the optional compound or compounds to be added to these compositions so that the advantageous properties intrinsically attached to the present invention are not, or not substantially, detrimentally affected by the envisaged addition.

Several examples of the preparation of active compounds of formula (I) according to the invention, along with results of biological activities of such compounds and various practical formulations based on these compounds.

### Example 1: 3-{3'-[((Methyl)(octanoyl)amino)methyl]biphenyl-4-yl)acrylic acid

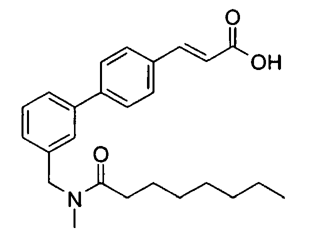

### a. N-Methyloctanamide

115 ml of triethylamine are added to a solution of 25 g (0.37 mol, 1 eq.) of methylamine hydrochloride in 125 ml of dichloromethane. 70 ml (0.41 mol, 1.1 eq.) of octanoyl chloride are slowly added at 0°C. The reaction mixture is stirred at ambient temperature for 3 hours and then filtered, and 100 ml of water are added to the filtrate. The organic phase is separated by settling, dried over sodium sulphate and evaporated. 61 g of N-methyloctanamide are obtained with a quantitative yield.

### b. N-Methyl-N-(3-bromobenzyl)octanamide

5 g (31.8 mmol, 1 eq) of N-methyloctanamide are added at 0°C to a suspension of 1.4 g (35 mmol, 1.1 eq.) of sodium hydride (60% in oil) in 60 ml of tetrahydrofuran. The reaction mixture is stirred at ambient temperature for 30 minutes and then a solution of 8.9 g (35 mmol, 1.1 eq.) of 3-bromobenzyl bromide in 15 ml of tetrahydrofuran is added. The mixture is stirred at ambient temperature for 16 hours. The reaction is halted by the addition of 100 ml of water and then extraction is carried out with ethyl acetate. The organic phases are combined and dried over sodium sulphate. The solvents are evaporated and then the residue is chromatographed on silica gel (heptane/ethyl acetate 75/25). 7.4 g of N-methyl-N-(3-bromobenzyl)octanamide are obtained. Yield = 71 %.

### c. Methyl[3-(4,4,5,5-tetramethyl-1,3,2-dioxaborolan-2-yl)benzy]octanamide

1.0 g (3.98 mmol, 1.3 eq.) of bis(pinacolato)diboron is added to a mixture of 1.0 g (3.06 mmol, 1 eq.) of N-methyl-N-(3-bromobenzyl)octanamide and of 900 mg (9.18 mmol, 3 eq.) of potassium acetate in 10 ml of dimethylformamide in the presence of 111 mg (0.15 mmol, 5 mol%) of [1,1'-bis(diphenylphosphino)ferrocene]dichloropalladium(II) (PdCl₂dppf). The mixture is stirred at 80°C for 2 hours. The reaction is halted by the addition of 20 ml of water and then extraction is carried out with ethyl acetate. The organic phases are combined and dried over sodium sulphate. The solvents are evaporated and then the residue is chromatographed on silica gel (heptane/ethyl acetate 80/20). 1.0 g of methyl[3-(4,4,5,5-tetramethyl-1,3,2-dioxaborolan-2-yl)benzyl]octanamide is obtained in the form of an oil. Yield = 88%

### d. Ethyl 3-(3'-[((methyl)(octanoyl)amino)methyl]biphenyl-4-yl}acrylate

30 mg (0.02 mmol, 5 mol%) of palladiumtetrakis(triphenylphosphine) are added to a solution of 200 mg (0.53 mmol, 1.0 eq.) of methyl[3-(4,4,5,5-tetramethyl-1,3,2-dioxaborolan-2-yl)benzyl]octanamide and 204 mg (0.80 mmol, 1.5 eq.) of ethyl 4-bromocinnamate in 3 ml of a mixture of dimethylformamide and of a 2M potassium phosphate solution (6/1). The reaction mixture is stirred at 80°C for 2 hours. The reaction is halted by the addition of 5 ml of water and then extraction is carried out with ethyl acetate. The organic phases are combined and dried over sodium sulphate. The solvents are evaporated and then the residue is chromatographed on silica gel (heptane/ethyl acetate 80/20). 145 mg of ethyl 3-(3'-[((methyl)(octanoyl)amino)methyl]biphenyl-4-yl}acrylate are obtained in the form of an oil. Yield = 63%

### e. 3-{3'-[((Methyl)(octanoyl)amino)methyl]biphenyl-4-yl)acrylic acid

300 mg (7.5 mmol, 22 eq.) of sodium hydroxide are added to a solution of 145 mg (0.34 mmol, 1 eq.) of ethyl 3-(3'-[((methyl)(octanoyl)amino)methyl]biphenyl-4-yl}acrylate in 3 ml of tetrahydrofuran/methanol (9/1). The reaction mixture is stirred at ambient temperature for 2 hours. The reaction is halted by the addition of 20 ml of water and 3 ml of acetic acid and then extraction is carried out with ethyl acetate. The organic phases are combined and dried over sodium sulphate. The solvents are evaporated and then the residue is crystallized from pentane/dichloromethane. 110 mg of 3-{3'-[((methyl)(octanoyl)amino)methyl]biphenyl-4-yl)acrylic acid are obtained in the form of a white powder.
(M.p. = 124-125°C) Yield = 78%
**¹H NMR** (CDCl₃, 400 MHz): 0.88 (m, 3H), 1.30 (m, 8H), 1.71 (m, 2H), 2.44 (t, *J* = 7.6 Hz, 2H), 2.99 & 3.03 (2s (rotamers), 3H), 4.65 & 4.70 (2s (rotamers), 2H), 6.51 (2d (rotamers), *J* = 15.9 Hz, 1H), 7.22 (2d (rotamers), *J* = 6.6 Hz, 1H), 7.41-7.66 (m, 7H), 7.82 (2d (rotamers), *J* = 15.9 Hz, 1 H).

### Example 2: 3-{3'-[((Methyl)(octanoyl)amino)methyl]biphenyl-4-yl}propanoic acid

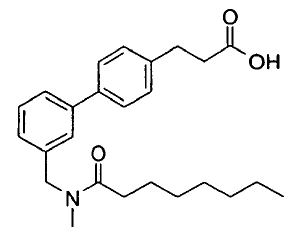

### a. Ethyl 3-{3'-[((methyl)(octanoyl)amino)methyl]biphenyl-4-yl}propanoate

A solution of 169 mg (0.40 mmol, 1 eq.) of ethyl 3-(3'-[((methyl)(octanoyl)amino)methyl]biphenyl-4-yl)acrylate (prepared as is 1d) in 2 ml of methanol in the presence of 50 mg of 10% palladium-on-charcoal is stirred at ambient temperature for 2 hours under a hydrogen atmosphere. The palladium is filtered off and then the solvents are evaporated. The residue is chromatographed on silica gel (heptane/ethyl acetate 80/20). 136 mg of ethyl 3-{3'-[((methyl)(octanoyl)amino)methyl]biphenyl-4-yl}propanoate are obtained in the form of an oil. Yield = 80%

### b. 3-{3-[((Methyl)(octanoyl)amino)methyl]biphenyl-4-yl}-propanoic acid

300 mg (7.5 mmol, 23 eq.) of sodium hydroxide are added to a solution of 136 mg (0.32 mmol, 1 eq.) of ethyl 3-{3'-[((methyl)(octanoyl)amino)methyl]biphenyl-4-yl}propanoate in 3 ml of tetrahydrofuran/methanol (9/1). The reaction mixture is stirred at ambient temperature for 2 hours. The reaction is halted by the addition of 20 ml of water and 3 ml of acetic acid and then the extraction is carried out with ethyl acetate. The organic phases are combined and dried over sodium sulphate. The solvents are evaporated and then the residue is crystallized from pentane/dichloromethane. 85 mg of 3-{3'-[((methyl)(octanoyl)amino)methyl]-biphenyl-4-yl}propanoic acid are obtained in the form of a white powder. (M.p. = 91°C) Yield = 65%
**¹H NMR** (CDCl₃, 400 MHz): 0.88 (m, 3H), 1.30 (m, 8H), 1.70 (m, 2H), 2.41 (t, J = 7.5 Hz, 2H), 2.74 (m, 2H), 2.97 & 3.00 (2s (rotamers), 3H), 3.01 (m, 2H), 4.61 & 4.68 (2s (rotamers), 2H), 6.51 (2d e, J = 15.9 Hz, 1H), 7.14 & 7.22 (2d (rotamers), J = 7.4 Hz, 1 H), 7.28-7.54 (m, 7H).

### Example 3: 3-{3-Fluoro-3'-[((methyl)(octanoyl)amino)methyl]biphenyl-4-yl}acrylic acid

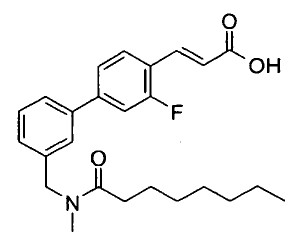

### a. Methyl 3-(4-bromo-2-fluorophenyl)acrylate

2.17 g (6.5 mmol, 1.2 eq.) of methyl (triphenylphosphoranylidene)acetate are added to a solution of 1.1 g (5.4 mmol, 1.0 eq.) of 4-bromo-2-fluorobenzaldehyde in 10 ml of toluene. The reaction mixture is stirred at 80°C for 1 hour. The reaction is halted by the addition of 20 ml of water and then extraction is carried out with ethyl acetate. The organic phases are combined and dried over sodium sulphate. The solvents are evaporated and then the residue is chromatographed on silica gel (heptane/ethyl acetate 80/20). 1.1 g of methyl 3-(4-bromo-2-fluorophenyl)acrylate are obtained in the form of a white solid.
Yield = 78%

### b. Methyl 3-{3-fluoro-3'-[((methyl)(octanoyl)amino)methyl]biphenyl}-4-yl}acrylate

500 mg (1.93 mmol, 1.0 eq.) of methyl 3-(4-bromo-2-fluorophenyl)acrylate and 721 mg (1.93 mmol, 1.0 eq.) of methyl[3-(4,4,5,5-tetramethyl-1,3,2-dioxaborolan-2-yl)benzyl]octanamide (prepared as in 1c) are dissolved in 12 ml of a mixture of dimethylformamide and of a 2M potassium phosphate solution (5/1). 111 mg (0.09 mmol, 5 mol%) of palladiumtetrakis(triphenylphosphine) are added and then the reaction mixture is stirred at 80°C for 2 hours. The reaction is halted by the addition of 30 ml of water and then extraction is carried out with ethyl acetate. The organic phases are combined and dried over sodium sulphate. The solvents are evaporated and then the residue is chromatographed on silica gel (heptane/ethyl acetate 80/20). 615 mg of methyl 3-{3-fluoro-3'-[((methyl)(octanoyl)amino)methyl]biphenyl-4-yl}acrylate are obtained. Yield = 75%

### c. 3-{3-Fluoro-3'-[((methyl)(octanoyl)amino]methyl]biphenyl-4-yl}acrylic acid

500 mg (12.5 mmol, 9 eq.) of sodium hydroxide are added to a solution of 615 mg (1.44 mmol, 1 eq.) of methyl 3-{3-fluoro-3'-[((methyl)(octanoyl)amino)methyl]biphenyl-4-yl}acrylate in 5 ml of tetrahydrofuran/methanol (9/1). The reaction mixture is stirred at ambient temperature for 2 hours. The reaction is halted by the addition of 20 ml of water and 3 ml of acetic acid and then extraction is carried out with ethyl acetate. The organic phases are combined and dried over sodium sulphate. The solvents are evaporated and then the residue is chromatographed on silica gel (dichloromethane/methanol 90/10). The oil obtained is crystallized from pentane. 510 mg of 3-{3-fluoro-3'-[((methyl)(octanoyl)amino)methyl]biphenyl-4-yl}acrylic acid are obtained in the form of a white powder. (M.p. = 91°C)
Yield = 86%
**¹H NMR** (CDCl₃, 400 MHz): 0.88 (m, 3H), 1.30 (m, 8H), 1.72 (m, 2H), 2.45 (m, 2H), 3.00 & 3.03 (2s (rotamers), 3H), 4.65 & 4.69 (2s (rotamers), 2H), 6.59 & 6.61 (2d rotamers, *J* = 16.1 Hz, 1H), 7.28-7.63 (m, 7H), 7.91 & 7.93 (2d rotamers, *J* = 16.1 Hz, 1H).

### Example 4: 3-{3-Fluoro-3'-[((methyl)(octanoyl)amino)methyl]biphenyl-4-yl}propanoic acid

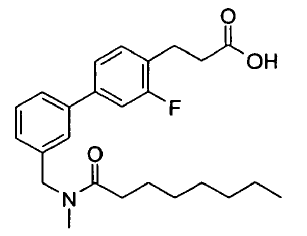

A solution of 200 mg (0.48 mmol, 1 eq.) of 3-{3-fluoro-3'-[((methyl)(octanoyl)amino)methyl]biphenyl-4-yl}acrylic acid (prepared as in 3c) in 3 ml of methanol in the presence of 50 mg of 10% palladium-on-charcoal is stirred at ambient temperature for 2 hours under a hydrogen atmosphere. The palladium is filtered off and then the solvents are evaporated. The residue is chromatographed on silica gel (dichloromethane/methanol 90/10). 154 mg of 3-{3-fluoro-3'-[((methyl)(octanoyl)amino)methyl]biphenyl-4-yl)propanoic acid are obtained. Yield = 78%
**¹H NMR** (CDCl₃, 400 MHz): 0.88 (m, 3H), 1.30 (m, 8H), 1.70 (m, 2H), 2.41 (t, *J* = 7.6 Hz, 2H), 2.74 (m, 2H), 2.97 & 3.00 (2s (rotamers), 3H), 3.05 (m, 2H), 4.62 & 4.67 (2s e, 2H), 7.18 & 7.49 (m, 7H).

### Example 5: 3-[3'-((Octanoylamino)methyl)biphenyl-4-yl]acrylic acid

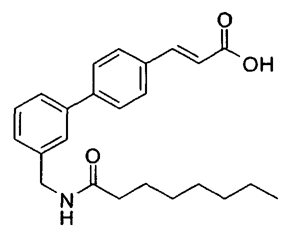

### a. Ethyl 3-[4-(4,4,5,5-tetramethyl-1,3.2-dioxaborolan-2-yl)ohenyl]acrylate

1.1 g (4.3 mmol, 1.1 eq.) of bis(pinacolato)diboron are added to a mixture of 1.0 g (3.9 mmol, 1 eq.) of ethyl 4-bromocinnamate and 1.1 g (11.7 mmol, 3 eq.) of potassium acetate in the presence of 142 mg (0.19 mmol, 5 mol%) of [1,1'-bis(diphenylphosphino)ferrocene]dichloropalladium(II) (PdCl₂dppf) in 10 ml of dimethylformamide. The mixture is stirred at 70°C for 2 hours. The reaction is halted by the addition of 20 ml of water and then extraction is carried out with ethyl acetate. The organic phases are combined and dried over sodium sulphate. The solvents are evaporated and then the residue is chromatographed on silica gel (heptane/ethyl acetate 90/10). 1.15 g of ethyl 3-[4-(4,4,5,5-tetramethyl-1,3,2-dioxaborolan-2-yl)phenyl]acrylate are obtained in the form of an oil. Yield = 98%

### b. Ethyl 3-[3'-((octanoylamino)methyl)biphenyl-4-yl]acrylate

124 mg (0.10 mmol, 5 mol%) of palladiumtetrakis(triphenylphosphine) are added to a solution of 500 mg (2.14 mmol, 1.0 eq.) of 3-iodobenzylamine and 712 mg (2.36 mmol, 1.1 eq) of ethyl 3-[4-(4,4,5,5-tetramethyl-1,3,2-dioxaborolan-2-yl)phenyl]acrylate in 6 ml of a mixture of dimethylformamide and of a 2M potassium phosphate solution (5/1). The reaction mixture is stirred at 80°C for 1 hour. The reaction is halted by the addition of 15 ml of water and then extraction is carried out with ethyl acetate. The organic phases are combined and dried over sodium sulphate. The solvents are evaporated and then the residue is dissolved in 25 ml of tetrahydrofuran. 2 ml of triethylamine are added, along with 0.5 ml (2.9 mmol, 1.4 eq.) of octanoyl chloride. The reaction mixture is stirred at ambient temperature for 1 hour. The reaction is halted by the addition of 20 ml of water and then extraction is carried out with ethyl acetate. The organic phases are combined and dried over sodium sulphate. The solvents are evaporated and then the residue is chromatographed on silica gel (heptane/ethyl acetate 70/30 down to 50/50). 430 mg of ethyl 3-[3'-((octanoylamino)methyl)biphenyl-4-yl]acrylate are obtained. Yield = 49%

### c. 3-[3'-((Octanoylamino)methyl)biphenyl-4-yl]acrylic acid

400 mg (10 mmol, 9 eq.) of sodium hydroxide are added to a solution of 430 mg (1.04 mmol, 1 eq.) of ethyl 3-[3'-((octanoylamino)methyl)biphenyl-4-yl]acrylate in 8 ml of tetrahydrofuran/methanol (9/1). The reaction mixture is stirred at ambient temperature for 2 hours. The reaction is halted by the addition of 20 ml of water and 3 ml of acetic acid and then extraction is carried out with ethyl acetate. The organic phases are combined and dried over sodium sulphate. The solvents are evaporated and then the residue is chromatographed on silica gel (dichloromethane/methanol 80/20). The oil obtained is crystallized from heptane/dichloromethane. 230 mg of 3-[3'-((octanoylamino)methyl)biphenyl-4-yl]acrylic acid are obtained in the form of a white powder. (M.p. = 176°C) Yield = 58%
**¹H NMR** (d₆-DMSO, 400 MHz): 0.83 (m, 3H), 1.24 (m, 8H), 1.53 (m, 2H), 2.15 (t, *J* = 7.6 Hz, 2H), 4.33 (d, *J* = 5.4 Hz, 2H), 6.58 (d, *J* = 15.9 Hz, 1H), 7.26 (d, *J* = 7.0 Hz, 1H), 7.42 (t, *J* = 7.8 Hz, 1H), 7.58 (m, 3H), 7.69 (d, *J* = 8.0 Hz, 2H), 7.78 (d, *J* = 7.7 Hz, 2H), 8.35 (s, 1H).

### Example 6: 3-[3'-((Octanoylamino)methyl)biphenyl-4-yl]propanoic acid

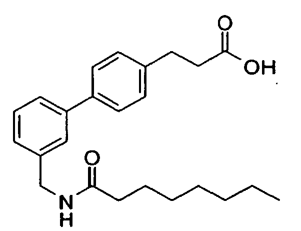

A solution of 100 mg (0.26 mmol, 1 eq.) of 3-[3'-((octanoylamino)methyl)biphenyl-4-yl]acrylic acid (prepared as in 5c) in 4 ml of methanol in the presence of 50 mg of 10% palladium-on-charcoal is stirred at ambient temperature for 6 hours under a hydrogen atmosphere. The palladium is filtered off and the solvents are evaporated. The residue is crystallized from dichloromethane/heptane and 65 mg of 3-[3'-((octanoylamino)methyl)biphenyl-4-yl]propanoic acid are obtained in the form of a white powder.
(M.p. = 124-125°C) Yield = 65%
**¹H NMR** (d₆-DMSO, 400 MHz): 0.84 (m, 3H), 1.24 (m, 8H), 1.53 (m, 2H), 2.14 (t, *J* = 6.7 Hz, 2H), 2.57 (t, *J* = 7.0 Hz, 2H), 2.86 (t, *J* = 6.7 Hz, 2H), 4.33 (d, *J* = 5 Hz, 2H), 7.21 (d, *J* = 6.9 Hz, 1H), 7.32 (d, *J* = 7.4 Hz, 2H), 7.39 (m, 1H), 7.53 (m, 4H), 8.33 (s, 1H), 12.11 (s, 1H).

### Example 7: 3-{3-Hydroxy-3'-[((methyl)(octanoyl)amino)methyl]biphenyl-4-yl}propanoic acid

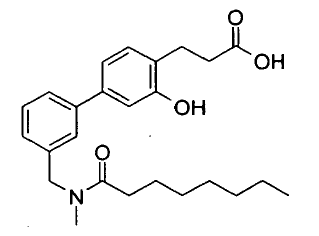

### a. Methyl 3-(2-benzyloxy-4-iodophenyl)acrylate

1.18 g (3.55 mmol, 1.2 eq.) of methyl (triphenylphosphoranylidene)acetate are added to a solution of 1.0 g (2.95 mmol, 1.0 eq.) of 2-benzyloxy-4-iodobenzaldehyde in 20 ml of toluene. The reaction mixture is stirred at 80°C for 2 hours. The reaction is halted by the addition of 20 ml of water and then extraction is carried out with ethyl acetate. The organic phases are combined and dried over sodium sulphate. The solvents are evaporated and then the residue is filtered through silica gel (heptane/ethyl acetate 50/50). The oil obtained is crystallized from pentane and 830 mg of methyl 3-(2-benzyloxy-4-iodophenyl)acrylate are obtained in the form of a white solid. Yield = 71 %

### b. Methyl 3-{3-benzyloxy-3'-[((methyl)(octanoyl)amino)methyl]biphenyl-4-yl}acrylate

500 mg (1.26 mmol, 1.0 eq.) of methyl 3-(2-benzyloxy-4-iodophenyl)acrylate and 474 mg (1.26 mmol, 1.0 eq.) of methyl[3-(4,4,5,5-tetramethyl-1,3,2-dioxaborolan-2-yl)benzyl]octanamide (prepared as in 1c) are dissolved in 6 ml of a mixture of dimethylformamide and of a 2M potassium phosphate solution (5/1). 72 mg (0.06 mmol, 5 mol%) of palladiumtetrakis(triphenylphosphine) are added and then the reaction mixture is stirred at 80°C for 2 hours. The reaction is halted by the addition of 30 ml of water and then extraction is carried out with ethyl acetate. The organic phases are combined and dried over sodium sulphate. The solvents are evaporated and then the residue is chromatographed on silica gel (heptane/ethyl acetate 80/20 down to 50/50). 530 mg of methyl 3-{3-benzyloxy-3'-[((methyl)(octanoyl)amino)-methyl]biphenyl-4-yl}acrylate are obtained. Yield = 82%

### c. 3-{3-Benzyloxy-3'-[((methyl)(octanoyl)amino)methyl]biphenyl-4-yl}acrylic acid

400 mg (10 mmol, 10 eq.) of sodium hydroxide are added to a solution of 530 mg (1.03 mmol, 1 eq.) of methyl 3-{3-benzyloxy-3'-[((methyl)(octanoyl)amino)methyl]biphenyl-4-yl}acrylate in 2 ml of tetrahydrofuran/methanol (9/1). The reaction mixture is stirred at ambient temperature for 2 hours. The reaction is halted by the addition of 20 ml of water and 3 ml of acetic acid and then extraction is carried out with ethyl acetate. The organic phases are combined and dried over sodium sulphate. The solvents are evaporated and then the residue is chromatographed on silica gel (dichloromethane/methanol 90/10). 405 mg of 3-{3-benzyloxy-3'-[((methyl)(octanoyl)amino)methyl]biphenyl-4-yl}acrylic acid are obtained. Yield = 79%

### d. 3-{3-Hydroxy-3'-[((methyl)(octanoyl)amino)methyl]biphenyl-4-yl}propanoic acid

A solution of 405 mg (0.81 mmol, 1 eq.) of 3-{3-benzyloxy-3'-[((methyl)(octanoyl)amino)methyl]biphenyl-4-yl}acrylic acid in 3 ml of methanol in the presence of 50 mg of 10% palladium-on-charcoal and of 20 µl of acetic acid is stirred at ambient temperature for 3 hours under a hydrogen atmosphere. The palladium is filtered off and then the solvents are evaporated. The residue is chromatographed on silica gel (dichloromethane/methanol 95/5). 135 mg of 3-{3-hydroxy-3'-[((methyl)(octanoyl)amino)methyl]biphenyl-4-yl}propanoic acid are obtained. Yield = 40%
**¹H NMR** (CDCl₃, 400 MHz): 0.86 (m, 3H), 1.28 (m, 8H), 1.69 (m, 2H), 2.41 (t, *J* = 7.7 Hz, 2H), 2.81 (m, 2H), 2.95 & 2.99 (2s (rotamers), 3H), 2.99 (m, 2H), 4.59 & 4.64 (2s e, 2H), 7.02-7.46 (m, 7H).

### Example 8: 3-{2-Butoxy-3'-[((methyl)(octanoyl)amino)methyl]biphenyl-4-yl}propanoic acid

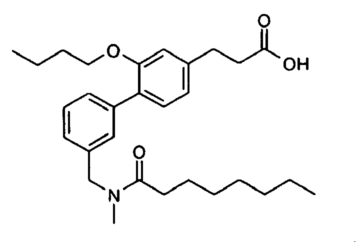

### a. 3-Hydroxy-4-iodobenzoic acid

21.0 g (0.52 mol, 1.05 eq.) of sodium hydroxide and then 78.7 g (0.52 mol, 1.05 eq.) of sodium iodide are added to a solution of 69.1 g (0.5 mol, 1 eq.) of 3-hydroxybenzoic acid in 700 ml of methanol. The reaction mixture is cooled to 0°C and then aqueous sodium hypochlorite solution (0.52 mol, 1.05 eq.) is added dropwise. The reaction medium is stirred at 0-5°C for 2 hours and then at ambient temperature overnight. The methanol is evaporated and then the reaction medium is acidified with a concentrated hydrochloric acid solution. The precipitated product is filtered off, washed with water and dried. 121 g of 3-hydroxy-4-iodobenzoic acid are obtained in the form of an off-white solid. Yield = 92%

### b. Methyl 3-hydroxy-4-iodobenzoate

59 ml (1.10 mol, 2.4 eq.) of sulphuric acid are added to a solution of 121 g (0.458 mol, 1 eq.) of 3-hydroxy-4-iodobenzoic acid in 700 ml of methanol. The reaction mixture is heated at reflux for 6 days. The methanol is evaporated and then the reaction medium is poured into water and extracted with ethyl acetate. The organic phases are combined, washed with water and dried over sodium sulphate. The solvent is concentrated and the solid obtained is filtered off and dried. 88.56 g of methyl-3-hydroxy-4-iodobenzoate are obtained in the form of white crystals. Yield = 70%

### c. Methyl 3-butoxy-4-iodobenzoate

In a manner analogous to Example 26b, by reaction of 21.5 ml (0.189 mol, 1.5 eq.) of 1-iodobutane and of 35.03 g (0.126 mol, 1 eq.) of methyl 3-hydroxy-4-iodobenzoate in 350 ml of methyl ethyl ketone in the presence of 52.24 g (0.378 mol, 3 eq.) of potassium carbonate at 85°C for 2 1/2 hours, 41.78 g of methyl 3-butoxy-4-iodobenzoate are obtained in the form of white crystals after washing with heptane. Yield = 99%

### d. (3-Butoxy-4-iodophenyl)methanol

8.17 g (0.375 mol, 3 eq.) of lithium borohydride are added to a solution of 41.78 g (0.125 mol, 1 eq.) of methyl 3-butoxy-4-iodobenzoate in 210 ml of tetrahydrofuran. The reaction medium is heated at 60°C for 2 hours and is then gently hydrolysed in an ice-cold and saturated ammonium chloride solution. The reaction medium is neutralized with concentrated hydrochloric acid and then extracted with ethyl acetate. The organic phases are washed with water and dried over magnesium sulphate. The solvent is evaporated and 38.31 g of (3-butoxy-4-iodophenyl)methanol are obtained in the form of a whitish oil. Yield = 100%

### e. 3-Butoxy-4-iodobenzaldehyde

89.5 g (0.875 mol, 7 eq.) of manganese dioxide are added to a solution of 38.30 g (0.125 mol, 1 eq.) of (3-butoxy-4-iodophenyl)methanol in 250 ml of dichloromethane. The reaction medium is stirred at ambient temperature for 18 hours and then filtered through silica gel. The solvent is evaporated and 29.61 g of 3-butoxy-4-iodobenzaldehyde are obtained in the form of an orange oil. Yield = 78%

### f. Methyl (E)-3-(3-butoxy-4-iodophenyl)acrylate

65.08 g (0.195 mol, 2 eq.) of methyl (triphenylphosphoranylidene)acetate are added to a solution of 29.60 g (0.097 mol, 1 eq.) of 3-butoxy-4-iodobenzaldehyde in 360 ml of toluene. The reaction mixture is heated at reflux for 2 hours. The solvent is evaporated and the oil obtained is chromatographed on silica gel (heptane/dichloromethane 50/50). 30.47 g of methyl (E)-3-(3-butoxy-4-iodophenyl)acrylate are obtained in the form of pale yellow crystals. Yield = 87%

### g. Methyl (E)-3-{2-butoxy-3'-[((methyl)(octanoyl)amino)methyl]biphenyl-4-yl}acrylate

4.5 mg (1 mol%) of palladium acetate and 14 mg (2 mol%) of biphenyldicyclohexylphosphine are added to a solution of 720 mg (2.0 mmol, 1 eq.) of methyl (E)-3-(3-butoxy-4-iodophenyl)acrylate (prepared in stage f) and of 971 mg (2.6 mmol, 1.3 eq.) of methyl [3-(4,4,5,5-tetramethyl-1,3,2-dioxoborolan-2-yl)benzyl]octanamide (prepared in Example 1c) in 12 ml of a mixture of dimethylformamide and of a 2M potassium phosphate solution (5/1). The mixture is heated at 90°C for 2 hours. The reaction medium is hydrolysed in an ammonium chloride solution and then extracted with ethyl acetate. The organic phases are combined, washed with water and dried over magnesium sulphate. The solvent is evaporated and then the residual oil is chromatographed on silica gel (heptane/ethyl acetate 75/25). 946 mg of methyl (E)-3-{2-butoxy-3'-[((methyl)(octanoyl)amino)methyl]biphenyl-4-yl}acrylate are obtained in the form of an orange-pink oil. Yield = 98%

### h. Methyl 3-{2-butoxy-3'-[((methyl)(octanoyl)amino)methyl]biphenyl-4-y/}propanoate

A solution of 916 mg (1.91 mmol, 1 eq.) of methyl (E)-3-{2-butoxy-3'-[((methyl)(octanoyl)amino)methyl]biphenyl-4-yl}acrylate in 10 ml of methanol in the presence of 92 mg (10% by weight) of 10% palladium-on-charcoal is stirred at ambient temperature for 3 1/2 hours under a hydrogen atmosphere. The palladium is filtered off through celite and then the solvent is evaporated. 902 mg of methyl 3-{2-butoxy-3'-[((methyl)(octanoyl)amino)methyl}biphenyl-4-yl}propanoate are obtained in the form of a yellowish oil. Yield = 98%

### i. 3-{2-Butoxy-3'-(methyl)(octanoyl)amino)methyl]biphenyl-4-yl}propanoic acid

740 mg (18.5 mmol, 10 eq.) of sodium hydroxide are added to a solution of 891 mg (1.85 mmol, 1 eq.) of methyl 3-{2-butoxy-3'-[((methyl)(octanoyl)amino)methyl]biphenyl-4-yl}propanoate in 20 ml of methanol. The reaction mixture is stirred at ambient temperature for 24 hours. The reaction medium is evaporated to dryness, the residue is taken up in water, acidification is carried out with a 2N hydrochloric acid solution and extraction is carried out with ethyl acetate. The organic phases are combined, washed with water and dried over magnesium sulphate. The solvent is evaporated and the oil obtained is chromatographed on silica gel (FlashSmart column 35 g) eluted with dichloromethane/methanol (98/2). 725 mg of 3-{2-butoxy-3'-[((methyl)(octanoyl)amino)methyl]biphenyl-4-yl}propanoic acid are obtained in the form of a whitish oil. Yield = 84%
**¹H NMR** (CDCl₃, 400 MHz): 0.86 (t, 3H), 0.91 (t, 3H), 1.24-1.40 (m, 10H), 1.67-1.70 (m, 4H), 2.39 (m, 2H), 2.72 (t, 2H), 2.93 (d, 3H), 2.96-3.0 (m, 2H), 3.92-3.96 (m, 2H), 4.56-4.63 (d, 2H), 6.82-6.87 (m, 2H), 7.10-7.20 (d, 1H), 7.23 (t, 1H), 7.33-7.46 (m, 3H).

### Example 9: 3-{2-Butoxy-3'-[((hexanoyl)(methyl)amino)methyl]biphenyl-4-yl}propanoic acid

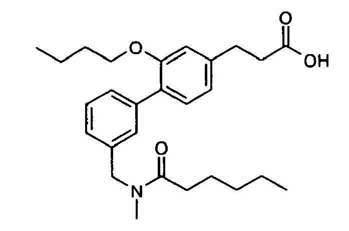

### a. tert-Butyl (3-bromobenzyl)carbamate

A solution of 25.0 g (0.11 mol, 1 eq.) of 3-bromobenzylamine hydrochloride and of 24.52 g (0.11 mol, 1 eq.) of di(tert-butyl) dicarbonate in 250 ml of dichloromethane in the presence of 15.6 ml (0.11mol, 1 eq.) of triethylamine is stirred at ambient temperature for 16 hours. The reaction medium is washed with water and separated by settling, and the organic phase is dried over sodium sulphate. The solvent is evaporated and 32.41 g of tert-butyl (3-bromobenzyl)carbamate are obtained in the form of crystals. Yield = 100%

### b. tert-Butyl (3-bromobenzyl)(methyl)carbamate

5.4 g (0.134 mol, 1.2 eq.) of 60% sodium hydride are added to a solution of 32.0 g (0.111 mol, 1 eq.) of tert-butyl (3-bromobenzyl)carbamate in 450 ml of dimethylformamide. The reaction medium is stirred at ambient temperature for 30 minutes and then 20.9 ml (0.335 mol, 3 eq.) of iodomethane are added. The reaction medium is stirred at ambient temperature for 20 hours, then hydrolysed in water and extracted with ethyl acetate. The organic phases are combined, washed with a saturated sodium chloride solution and dried over sodium sulphate. The solvent is evaporated and 36.23 g of tert-butyl (3-bromobenzyl)(methyl)carbamate are obtained in the form of an orange oil. Yield = 100%

### c. tert-Butyl methyl[3-(4,4,5,5-tetramethyl-1,3,2-dioxaborolan-2-yl)benzyl]carbamate

In a manner analogous to Example 1c, by reaction of 33.0 g (0.11 mol, 1 eq.) of tert-butyl (3-bromobenzyl)(methyl)carbamate, of 29.3 g (0.115 mol, 1.05 eq.) of bis(pinacolato)diboron and of 32.3 g (0.33 mol, 3 eq.) of potassium acetate in 500 ml of dimethylformamide in the presence of 3.6 g (4 mol%) of [1,1'-bis(diphenylphosphino)ferrocene]dichloropalladium(II) (PdCl₂dppf), 31.56 g of tert-butyl methyl[3-(4,4,5,5-tetramethyl-1,3,2-dioxaborolan-2-yl)benzyl]carbamate are obtained, after chromatography on silica gel (heptane/ethyl acetate 90/10), in the form of a green oil. Yield = 83%

### d. Methyl (E)-3-{2-butoxy-3'-[((tert-butoxycarbonyl)(methyl)amino)methyl]biphenyl-4-yl}acrylate

In a manner analogous to Example 8g, by reaction of 3.6 g (10.0 mmol, 1 eq.) of methyl (E)-3-(3-butoxy-4-iodophenyl)acrylate (prepared in Example 8f) and of 4.51 g (13 mmol, 1.3 eq.) of tert-butyl methyl[3-(4,4,5,5-tetramethyl-1,3,2-dioxaborolan-2-yl)benzyl]carbamate in 60 ml of a mixture of dimethylformamide and of a 2M potassium phosphate solution (5/1) in the presence of 22 mg (1 mol%) of palladium acetate and of 14 mg (2 mol%) of biphenyldicyclohexylphosphine, 3.87 g of methyl (E)-3-{2-butoxy-3'-[((tert-butoxycarbonyl)(methyl)amino)methyl]biphenyl-4-yl}acrylate are obtained in the form of a yellowish oil. Yield = 85%

### e. Methyl 3-{2-butoxy-3'-[((tert-butoxycarbonyl)(methyl)amino)methyl]biphenyl-4-yl}propanoate

In a manner analogous to Example 8h, by reaction of 3.86 g (8.51 mmol, 1 eq.) of methyl (E)-3-{2-butoxy-3'-[((tert-butoxycarbonyl)(methyl)amino)-methyl]biphenyl-4-yl}crylate in 10 ml of methanol in the presence of 386 mg (10% by weight) of 10% palladium-on-charcoal, 3.32 g of methyl 3-{2-butoxy-3'-[((tert-butoxycarbonyl)(methyl)amino)methyl]biphenyl-4-yl}propanoate are obtained in the form of a colourless oil. Yield = 86%

### f. Methyl 3-(2-butoxy-3'-(methylaminomethyl)biphenyl-4-yl)propanoate

A solution of 3.31 g (7.27 mmol, 1 eq.) of methyl 3-{2-butoxy-3'-[((tert-butoxycarbonyl)(methyl)amino)methyl]biphenyl-4-yl}propanoate in 40 ml of dichloromethane in the presence of 5.4 ml (72.7 mmol, 10 eq.) of trifluoroacetic acid is stirred at ambient temperature for 2 hours. The solvents are evaporated and the oil obtained is chromatographed on silica gel (FlashSmart column 120 g) eluted with dichloromethane/methanol (97/3). 3.32 g of methyl 3-(2-butoxy-3'-(methylaminomethyl)biphenyl-4-yl)propanoate are obtained in the form of a whitish oil. Yield = 100%

### g. Methyl 3-{2-butoxy-3'-[((hexanoyl)(methyl)amino)methyl]biphenyl-4-yl}propanoate

A solution of 480 mg (1.35 mmol, 1 eq.) of methyl 3-(2-butoxy-3'-(methylaminomethyl)biphenyl-4-yl)propanoate and of 377 µl (2.70 mmol, 2 eq.) of hexanoyl chloride in 15 ml of dichloromethane in the presence of 560 µl (4.05 mmol, 3 eq.) of triethylamine and of 16 mg (10 mol%) of 4-dimethylaminopyridine is stirred at ambient temperature for 16 hours. The reaction medium is hydrolysed with a 2N hydrochloric acid solution and separated by settling. The organic phase is washed with water and dried over magnesium sulphate. The solvent is evaporated and the oil obtained is chromatographed on silica gel (FlashSmart column 35 g) eluted with heptane/ethyl acetate (70/30). 400 mg of methyl 3-{2-butoxy-3'-[((hexanoyl)(methyl)amino)methyl]biphenyl-4-yl}propanoate are obtained in the form of an oil. Yield = 65%

### h. 3-{2-Butoxy-3'-[((hexanoyl)(methyl)amino)methyl]biphenyl-4-y/}propanoic acid

A solution of 390 mg (0.86 mmol, 1 eq.) of methyl 3-{2-butoxy-3'-[((hexanoyl)(methyl)amino)methyl]biphenyl-4-yl}propanoate and of 4.3 ml (4.3 mmol, 5 eq.) of a 1N lithium hydroxide solution in 10 ml of tetrahydrofuran is stirred at ambient temperature for 20 hours. The reaction medium is concentrated, acidified with a 2N hydrochloric acid solution and then extracted with ethyl acetate. The organic phases are combined, washed with water and dried over magnesium sulphate. The solvent is evaporated and the oil obtained is chromatographed on silica gel (FlashSmart column 20 g) eluted with dichloromethane/methanol (98/2). 378 mg of 3-{2-butoxy-3'-[((hexanoyl)(methyl)amino)methyl]biphenyl-4-yl}propanoic acid are obtained in the form of a yellowish oil.
Yield = 100%
**¹H NMR** (CDCl₃, 400 MHz): 0.86-0.91 (m, 6H), 1.28-1.41 (m, 6H), 1.65-1.70 (m, 4H), 2.38 (m, 2H), 2.72 (t, 2H), 2.93 (d, 3H), 2.96-3.0 (m, 2H), 3.92-3.96 (m, 2H), 4.56-4.63 (d, 2H), 6.82-6.87 (m, 2H), 7.05-7.15 (d, 1H), 7.22 (t, 1H), 7.33-7.46 (m, 3H).

### Example 10: 3-{2-Butoxy-3'-[((methyl)(pentanoyl)amino)methyl]biphenyl-4-yl}propanoic acid

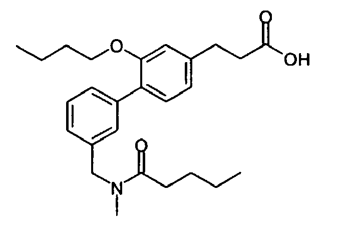

### a. Methyl 3-{2-butoxy-3'-[((methyl)(pentanoyl)amino)methyl]biphenyl-4-yl}propanoate

In a manner analogous to Example 9g, by reaction of 480 mg (1.35 mmol, 1 eq.) of methyl 3-(2-butoxy-3'-(methylaminomethyl)biphenyl-4-yl)propanoate (prepared in Example 9f) and of 327 µl (2.70 mmol, 2 eq.) of pentanoyl chloride in 15 ml of dichloromethane in the presence of 560 µl (4.05 mmol, 3 eq.) of triethylamine and of 16 mg (10 mol%) of 4-dimethylaminopyridine, 390 mg of methyl 3-{2-butoxy-3'-[((methyl)(pentanoyl)amino)methyl]biphenyl-4-yl}propanoate are obtained in the form of an oil. Yield = 66%

### b. 3-{2-Butoxy-3'-[((methyl)(pentanoyl)amino)methyl]biphenyl-4-yl}propanoic acid

In a manner analogous to Example 9h, by reaction of 380 mg (0.864 mmol, 1 eq.) of methyl 3-{2-butoxy-3'-[((methyl)(pentanoyl)amino)-methyl]biphenyl-4-yl}propanoate and of 4.3 ml (4.3 mmol, 5 eq.) of a 1N lithium hydroxide solution in 10 ml of tetrahydrofuran, 361 mg of 3-{2-butoxy-3'-[((methyl)(pentanoyl)amino)methyl]biphenyl-4-yl}propanoic acid are obtained in the form of a yellowish oil. Yield = 98%
**¹H NMR** (CDCl₃, 400 MHz): 0.87-0.96 (m, 6H), 1.35-1.42 (m, 4H), 1.65-1.70 (m, 4H), 2.38 (m, 2H), 2.73 (t, 2H), 2.94 (d, 3H), 2.98-3.1 (m, 2H), 3.93-3.96 (m, 2H), 4.56-4.63 (d, 2H), 6.82-6.87 (m, 2H), 7.05-7.15 (d, 1H), 7.22 (t, 1H), 7.33-7.44 (m, 3H).

### Example 11: 3-{2-Butoxy-3'-[((heptanoyl)(methyl)amino)methyl]biphenyl-4-yl}propanoic acid

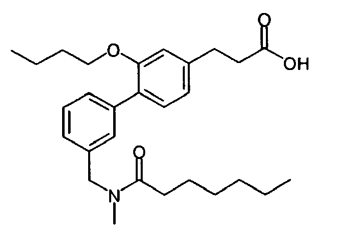

### a. Methyl 3-{2-butoxy-3'-[((heptanoyl)(methyl)amino)methyl]biphenyl-4-yl}propanoate

In a manner analogous to Example 9g, by reaction of 480 mg (1.35 mmol, 1 eq.) of methyl 3-(2-butoxy-3'-(methylaminomethyl)biphenyl-4-yl)propanoate (prepared in Example 9f) and of 418 µl (2.70 mmol, 2 eq.) of heptanoyl chloride in 15 ml of dichloromethane in the presence of 560 µl (4.05 mmol, 3 eq.) of triethylamine and of 16 mg (10 mol%) of 4-dimethylaminopyridine, 410 mg of methyl 3-{2-butoxy-3'-[((heptanoyl)(methyl)amino)methyl]biphenyl-4-yl}propanoate are obtained in the form of an oil. Yield = 65%

### b. 3-{2-Butoxy-3'-[((heptanoyl)(methyl)amino)methyl]biphenyl-4-yl}propanoic acid

In a manner analogous to Example 9h, by reaction of 400 mg (0.855 mol, 1 eq.) of methyl 3-{2-butoxy-3'-[((heptanoyl)(methyl)amino)-methyl]biphenyl-4-yl}propanoate and of 4.3 ml (4.3 mmol, 5 eq.) of a 1N lithium hydroxide solution in 10 ml of tetrahydrofuran, 378 mg of 3-{2-butoxy-3'-[((heptanoyl)(methyl)amino)methyl]biphenyl-4-yl}propanoic acid are obtained in the form of a yellowish oil. Yield = 97.5%
**¹H NMR** (CDCl₃, 400 MHz): 0.85-0.93 (m, 6H), 1.26-1.41 (m, 8H), 1.67-1.70 (m, 4H), 2.39 (m, 2H), 2.73 (m, 2H), 2.94 (d, 3H), 2.98-3.01 (m, 2H), 3.93-3.96 (m, 2H), 4.56-4.63 (d, 2H), 6.82-6.87 (m, 2H), 7.05-7.15 (d, 1H), 7.22 (t, 1H), 7.33-7.44 (m, 3H).

### Example 12: 3-(2-Butoxy-31-{[(4-ethoxybenzoyl)(methyl)-amino]methyl}biphenyl-4-yl)propanoic acid

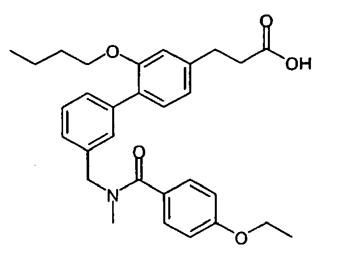

### a. Methyl 3-(2-butoxy-3'-{[(4-ethoxvbenzoyl)(methyl)amino]methyl}biphenyl-4-yl)propanoate

In a manner analogous to Example 9g, by reaction of 400 mg (1.12 mmol, 1 eq.) of methyl 3-(2-butoxy-3'-(methylaminomethyl)biphenyl-4-yl)propanoate (prepared in Example 9f) and of 220 µl (1.40 mmol, 1.2 eq.) of 4-ethoxybenzoyl chloride in 10 ml of dichloromethane in the presence of 500 µl (3.58 mmol, 3.2 eq.) of triethylamine at ambient temperature for 12 hours, methyl 3-(2-butoxy-3'-{[(4-ethoxybenzoyl)(methyl)amino]methyl}biphenyl-4-yl)propanoate is obtained in the form of an oil which is used as is in the following reaction.

### b. 3-(2-Butoxy-3'-{[(4-ethoxybenzoyl)(methyl)amino]methyl}biphenyl-4-yl)propanoic acid

In a manner analogous to Example 1e, by reaction of 400 mg (10 mmol) of sodium hydroxide and of the methyl 3-(2-butoxy-3'-{[(4-ethoxybenzoyl)(methyl)amino]methyl}biphenyl-4-yl)propanoate prepared in the preceding stage in 10 ml of tetrahydrofuran/methanol (8/2), 360 mg of 3-(2-butoxy-3'-{[(4-ethoxybenzoyl)(methyl)amino]methyl}biphenyl-4-yl)propanoic acid are obtained in the form of a gum after chromatography on silica gel (heptane/ethyl acetate 70/30). Yield = 66% over the 2 stages.
**¹H NMR** (CDCl₃, 400 MHz): 0.88 (t, 3H), 1.35-1.43 (m & t, 5H), 1.64-1.69 (m, 2H), 2.68 (t, 2H), 2.98 (t & m, 5H), 3.94 (t, 2H), 4.01-4.06 (m, 2H), 4.60 & 4.75 (2m (rotamers), 2H), 6.81 (s, 1H), 6.82-6.87 (m, 3H), 7.10-7.24 (m, 2H), 7.38 (t, 1H), 7.43-7.46 (m, 4H).

### Example 13: 3-(2-Butoxy-3'-{[(4-butoxybenzoyl)methyl)-aminolmethyl}biphenyl-4-yl)propanoic acid

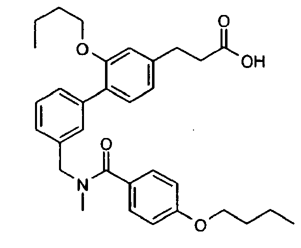

### a. Methyl 3-(2-butoxy-3'-{[(4-butoxybenzoyl)(methyl)amino]methyl}biphenyl-4-y/)propanoate

In a manner analogous to Example 9g, by reaction of 400 mg (1.12 mmol, 1 eq.) of methyl 3-(2-butoxy-3'-(methylaminomethyl)biphenyl-4-yl)propanoate (prepared in Example 9f) and of 250 µl (1.40 mmol, 1.2 eq.) of 4-butoxybenzoyl chloride in 10 ml of dichloromethane in the presence of 500 µl (3.58 mmol, 3.2 eq.) of triethylamine at ambient temperature for 12 hours, methyl 3-(2-butoxy-3'-{[(4-butoxybenzoyl)(methyl)amino]methyl}biphenyl-4-yl)propanoate is obtained in the form of an oil which is used as is in the following reaction.

### b. 3-(2-Butoxy-3'-{[(4-butoxybenzoy/)(methyl)amino]methyl}biphenyl-4-yl)propanoic acid

In a manner analogous to Example le, by reaction of 400 mg (10 mmol) of sodium hydroxide and of the methyl 3-(2-butoxy-3'-{[(4-butoxybenzoyl)(methyl)amino]methyl}biphenyl-4-yl)propanoate prepared in the preceding stage in 10 ml of tetrahydrofuran/methanol (8/2), 345 mg of 3-(2-butoxy-3'-{[(4-butoxybenzoyl)(methyl)amino]methyl}biphenyl-4-yl)propanoic acid are obtained in the form of a gum after chromatography on silica gel (heptane/ethyl acetate 70/30). Yield = 60% over the 2 stages.
**¹H NMR** (CDCl₃, 400 MHz): 0.88 (t, 3H), 0.97 (t, 3H), 1.35-1.40 (m, 2H), 1.45-1.51 (m, 2H), 1.64-1.69 (m, 2H), 1.74-1.78 (m, 2H), 2.68 (t, 2H), 2.98 (t & m, 5H), 3.93-3.98 (m, 4H), 4.60 & 4.75 (2m e, 2H), 6.82 (s, 1H), 6.84-6.88 (m, 3H), 7.10-7.25 (m, 2H), 7.38 (t, 1H), 7.42-7.46 (m, 4H).

### Example 14: 3-{3'-[((Benzoyl)methyl)amino)methyl]-2-butoxybiphenyl-4-yl}propanoic acid

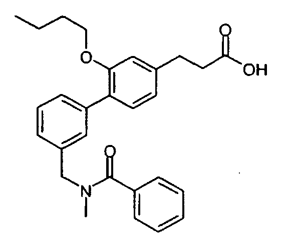

### a. Methyl 3-{3'-[((benzoyl)(methyl)amino)methyl]-2-butoxybiphenyl-4-yl}propanoate

In a manner analogous to Example 9g, by reaction of 500 mg (1.4 mmol, 1 eq.) of methyl 3-(2-butoxy-3'-(methylaminomethyl)biphenyl-4-yl)propanoate (prepared in Example 9f) and of 325 µl (2.80 mmol, 2 eq.) of benzoyl chloride in 10 ml of dichloromethane in the presence of 600 µl (4.3 mmol, 3 eq.) of triethylamine and of 17 mg (10 mol%) of 4-dimethylaminopyridine, 573 mg of methyl 3-{3'-[((benzoyl)(methyl)amino)methyl]-2-butoxybiphenyl-4-yl}propanoate are obtained in the form of a yellow oil. Yield = 89%

### b. 3-{3'-[((Benzoyl)(methyl)amino)methyl]-2-butoxybiphenyl-4-yl}propanoic acid

493 mg (12.3 mmol, 10 eq.) of sodium hydroxide are added to a solution of 566 mg (1.23 mmol, 1 eq.) of methyl 3-{3'-[((benzoyl)(methyl)amino)methyl]-2-butoxybiphenyl-4-yl}propanoate in 10 ml of methanol. The reaction mixture is heated at 50°C for 2 hours. The reaction medium is evaporated to dryness, the residue is taken up in water, acidification is carried out with a 1 N hydrochloric acid solution and extraction is carried out with ethyl acetate. The organic phases are combined, washed with water and dried over magnesium sulphate. The solvent is evaporated and the product is crystallized in an ethyl ether/heptane mixture. 450 mg of 3-{3'-[((benzoyl)(methyl)amino)methyl]-2-butoxybiphenyl-4-yl}propanoic acid are obtained in the form of a white powder. (M.p. = 103-105°C) Yield = 82%
**¹H NMR** (CDCl₃, 400 MHz): 0.87 (t, 3H), 1.38 (m, 2H), 1.64-1.70 (m, 2H), 2.73 (t, 2H), 2.88 & 3.06 (2s e, 3H), 2.99 (t, 2H), 3.95 (t, 2H), 4.54 & 4.80 (2s (rotamers), 2H), 6.83 (s, 1H), 6.87 (d, 1H), 7.09-7.51 (m, 10H).

### Example 15: 3-(2-Butoxy-3'-{[(4-methoxybenzoyl)(methyl)amino]methyl}biphenyl-4-yl)propanoic acid

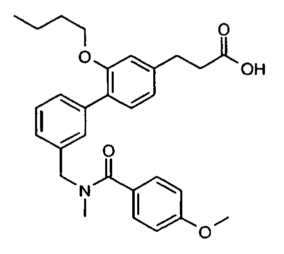

### a. Methyl 3-(2-butoxy-3'-{[(4-methoxybenzoyl)(methyl)amino]methyl}biphenyl-4-yl)propanoate

In a manner analogous to Example 9g, by reaction of 500 mg (1.4 mmol, 1 eq.) of methyl 3-(2-butoxy-3'-(methylaminomethyl)biphenyl-4-yl)propanoate (prepared in Example 9f) and of 386 µl (2.80 mmol, 2 eq.) of 4-methoxybenzoyl chloride in 10 ml of dichloromethane in the presence of 600 µl (4.3 mmol, 3 eq.) of triethylamine and of 17 mg (10 mol%) of 4-dimethylaminopyridine, 623 mg of methyl 3-(2-butoxy-3'-{[(4-methoxybenzoyl)(methyl)amino]methyl}biphenyl-4-yl)propanoate are obtained in the form of a yellow oil. Yield = 91%

### b. 3-(2-Butoxy-3'-{[(4-methoxybenzoyl)(methyl)amino]methyl}biphenyl-4-yl)propanoic acid

In a manner analogous to Example 14b, by reaction of 503 mg (12.6 mmol, 10 eq.) of sodium hydroxide and of 616 mg (1.26 mmol, 1 eq.) of methyl 3-(2-butoxy-3'-{[(4-methoxybenzoyl)(methyl)amino]methyl}biphenyl-4-yl)propanoate, prepared in the preceding stage, in 10 ml of methanol, 470 mg of 3-(2-butoxy-3'-{[(4-methoxybenzoyl)(methyl)amino]methyl}biphenyl-4-yl)propanoic acid are obtained in the form of an amorphous solid. (M.p. = 39-41°C) Yield = 79%
**¹H NMR** (CDCl₃, 400 MHz): 0.87 (t, 3H), 1.33-1.42 (m, 2H), 1.64-1.71 (m, 2H), 2.73 (t, 2H), 2.99 (t & m, 5H), 3.81 (s, 3H), 3.95 (t, 2H), 4.60 & 4.75 (2m e, 2H), 6.83-6.89 (m, 4H), 7.23-7.46 (m, 7H).

### Example 16: 3-(2-Butoxy-3'-{[(3-methoxybenzoyl)(methyl)amino]methyl}biphenyl-4-yl)propanoic acid

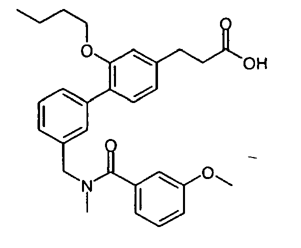

### a. Methyl 3-(2-butoxy-3'-{[(3-methoxybenzoyl)(methyl)amino]methyl}biphenyl-4-yl)propanoate

In a manner analogous to Example 9g, by reaction of 500 mg (1.4 mmol, 1 eq.) of methyl 3-(2-butoxy-3'-(methylaminomethyl)biphenyl-4-yl)propanoate (prepared in Example 9f) and of 393 µl (2.80 mmol, 2 eq.) of 3-methoxybenzoyl chloride in 10 ml of dichloromethane in the presence of 600 µl (4.3 mmol, 3 eq.) of triethylamine and of 17 mg (10 mol%) of 4-dimethylaminopyridine, 583 mg of methyl 3-(2-butoxy-3'-{[(3-methoxybenzoyl)(methyl)amino]methyl}biphenyl-4-yl)propanoate are obtained in the form of a yellow oil. Yield = 85%

### b. 3-(2-Butoxy-3'-{[(3-methoxybenzoyl)(methyl)amino]methyl}biphenyl-4-yl)propanoic acid

In a manner analogous to Example 14b, by reaction of 471 mg (11.8 mmol, 10 eq.) of sodium hydroxide and of 576 mg (1.18 mmol, 1 eq.) of methyl 3-(2-butoxy-3'-{[(3-methoxybenzoyl)(methyl)amino]methyl}biphenyl-4-yl)propanoate, prepared in the preceding stage, in 10 ml of methanol, 384 mg of 3-(2-butoxy-3'-{[(3-methoxybenzoyl)(methyl)amino]methyl}biphenyl-4-yl)propanoic acid are obtained in the form of a white powder. (M.p. = 80-82°C) Yield = 67%
**¹H NMR** (CDCl₃, 400 MHz): 0.88 (t, 3H), 1.37 (m, 2H), 1.67 (m, 2H), 2.73 (t, 2H), 2.88 & 3.07 (2s e, 3H), 2.99 (t, 2H), 3.69 & 3.82 (2s (rotamer, 3H), 3.94 (t, 2H), 4.53 & 4.79 (2s (rotamers), 2H), 6.83 (s, 1H), 6.86 (d, 1H), 6.93-7.22 (m, 4H), 7.30-7.50 (m, 5H).

### EXAMPLE 17: CROSSED-CURVE PPAR TRANSACTIVATION ASSAY

Activation of the PPAR receptors by an agonist (activator) in HeLN cells leads to the expression of a reporter gene, luciferase, which, in the presence of a substrate, generates light. The modulation of the PPAR receptors is measured by quantifying the luminescence produced after incubation of the cells in the presence of a reference agonist. The ligands will displace the agonist from its site. The measurement of the activity is performed by quantifying the light produced. This measurement makes it possible to determine the modulatory activity of the compounds according to the invention by the determination of the constant which represents the affinity of the molecule for the PPAR receptor. Since this value can fluctuate depending on the basal activity and the expression of the receptor, it is referred to as apparent Kd (Kdapp in nM).

To determine this constant, "crossed curves" for the test product, against a reference agonist, are prepared using a 96-well plate: 10 concentrations of the test product plus a concentration 0 are arranged in a line, and 7 concentrations of the agonist plus a concentration 0 are arranged in a column. This represents 88 measurement points for 1 product and 1 receptor. The remaining 8 wells are used for repeatability controls.

In each well, the cells are in contact with a concentration of the test product and a concentration of the reference agonist, 2-(4-{2-[3-(2,4-difluorophenyl)-1-heptylureido]ethyl}phenylsulphanyl)-2-methylpropionic acid for PPARα, {2-methyl-4-[4-methyl-2-(4-(trifluoromethyl)phenyl)thiazol-5-ylmethylsulphanyl]-phenoxy}acetic acid for PPARδ and 5-{4-[2-(methyl(pyrid-2-yl)amino)ethoxy]benzyl}thiazolidine-2,4-dione for PPARγ. Measurements are also taken for total agonist controls with the same products.

The HeLN cell lines used are stable transfectants containing the plasmids ERE-βGlob-Luc-SV-Neo (reporter gene) and PPAR (α, δ, γ) Gal-hPPAR. These cells are seeded in 96-well plates at the rate of 10 000 cells per well in 100 µl of DMEM medium without phenol red and supplemented with 10% of defatted calf serum. The plates are then incubated for 16 hours at 37°C and 7% CO₂.

The various dilutions of the test products and of the reference ligand are added at the rate of 5 µl per well. The plates are subsequently incubated for 18 hours at 37°C and 7% CO₂. The culture medium is removed by turning over and 100 µl of a 1:1 PBS/luciferin mixture are added to each well. After 5 minutes, the plates are read using the luminescence reader.

These crossed curves make it possible to determine the AC50 values (concentration at which 50% activation is observed) of the reference ligand at various concentrations of test product. These AC50 values are used to calculate the Schild regression by plotting a straight line corresponding to the Schild equation *("*Quantitation in Receptor Pharmacology", Terry P. Kenakin, Receptors and Channels, 2001, 7, 371-385) which allows the Kdapp values (in nM) to be obtained.

### Transactivation results:

| Compounds | PPARa Kdapp (in nM) | PPARd Kdapp (in nM) | PPARγ Kdapp (in nM) |
|---|---|---|---|
| Reference 1: 2-(4-{2-[3-(2,4-difluorophenyl)-1-heptylureido]ethyl}phenylsulphanyl)-2-methyl propionic acid | 200 | n.a. | n.a. |
| Reference 2: {2-methyl-4-[4-methyl-2-(4-(trifluoromethyl)phenyl)thiazol-5-ylmethylsulphanyl]phenoxy}acetic acid | n.a. | 10 | n.a. |
| Reference 3: 5-{4-[2-(methyl(pyridin-2-yl)amino)ethoxy]benzyl}thiazolidine-2,4-dione | n.a. | n.a. | 30 |
| Example 1 | n.a. | n.a. | 500 |
| Example 2 | n.a. | n.a. | 120 |
| Example 3 | n.a. | n.a. | 60 |
| Example 4 | n.a. | n.a. | 250 |
| Example 6 | n.a. | n.a. | 4000 |
| Example 7 | n.a. | n.a. | 4000 |
| Example 8 | - | - | 1 |
| Example 9 | 4000 | 4000 | 4 |
| Example 10 | 8000 | 4000 | 4 |
| Example 11 | 500 | 4000 | 1 |
| Example 12 | 8000 | 8000 | 0.25 |
| Example 13 | 8000 | 8000 | 1 |
| Example 14 | n.a. | 8000 | 8 |
| Example 15 | n.a. | 4000 | 0.5 |
| Example 16 | n.a. | 4000 | 4 |

| | | | |
|---|---|---|---|
| n.a. means not active | | | |

These results show the affinity of the compounds for PPAR receptors and more particularly the specificity of the affinity of the compounds of the invention for the PPARγ subtype, compared with the affinity of the compounds for the PPARα subtype or for the PPARδ subtype.

### EXAMPLE 18: COMPOSITIONS

Various practical formulations based on the compounds according to the invention are illustrated in this example.

### A- ORAL ROUTE

(a) 0.2 g tablet
   - Compound of Example 1 0.001 g
   - Starch 0.114 g
   - Dicalcium phosphate 0.020 g
   - Silica 0.020 g
   - Lactose 0.030 g
   - Talc 0.010 g
   - Magnesium stearate 0.005 g
(b) Suspension to be taken orally in 5 ml vials
   - Compound of Example 5 0.001 g
   - Glycerol 0.500 g
   - 70% Sorbitol 0.500 g
   - Sodium saccharinate 0.010 g
   - Methyl para-hydroxybenzoate 0.040 g
   - Flavouring q.s.
   - Purified water q.s. for 5 ml
(c) 0.2 g tablet
   - Compound of Example 2 0.050 g
   - Lactose monohydrate 0.132 g
   - Crospovidone 0.007 g
   - Povidone 0.005 g
   - Aerosil 2000.004 g
   - Magnesium stearate 0.002 g
(d) Suspension to be taken orally in 10 ml vials
   - Compound of Example 4 0.200 g
   - Glycerol 1.000 g
   - 70% Sorbitol 1.000 g
   - Sodium saccharinate 0.010 g
   - Methyl para-hydroxybenzoate 0.080 g
   - Flavouring q.s.
   - Purified water q.s. for 10 ml

### B- TOPICAL ROUTE

(a) Salve
   - Compound of Example 6 0.020 g
   - Isopropyl myristate 81.700 g
   - Liquid petrolatum 9.100 g
   - Silica ("Aerosil 200", sold by Degussa) 9.180 g
(b) Salve
   - Compound of Example 2 0.300 g
   - White petrolatum,
      pharmaceutical grade q.s. for 100 g
(c) Nonionic water-in-oil cream
   - Compound of Example 1 0.100 g
   - Mixture of emulsive lanolin alcohols, of waxes and of oils ("Anhydrous eucerin", sold by BDF) 39.900 g
   - Methyl para-hydroxybenzoate 0.075 g
   - Propyl para-hydroxybenzoate 0.075 g
   - Sterile demineralized water q.s. for 100 g
(d) Lotion
   - Compound of Example 3 0.100 g
   - Polyethylene glycol (PEG) 400 69.900 g
   - 95% Ethanol 30.000 g
(e) Hydrophobic salve
   - Compound of Example 5 0.300 g
   - Isopropyl myristate 36.400 g
   - Silicone oil ("Rhodorsil 47 V 300", sold by Rhône-Poulenc) 36.400 g
   - Beeswax13.600 g
   - Silicone oil ("Abil 300,000 cSt", sold by Goldschmidt) q.s. for 100 g
(f) Nonionic oil-in-water cream
   - Compound of Example 2 1.000 g
   - Cetyl alcohol 4.000 g
   - Glyceryl monostearate 2.500 g
   - PEG 50 stearate 2.500 g
   - Shea butter 9.200 g
   - Propylene glycol 2.000 g
   - Methyl para-hydroxybenzoate 0.075 g
   - Propyl para-hydroxybenzoate 0.075 g
   - Sterile demineralized water q.s. for 100 g

## Claims

1. Compounds, **characterized in that** they correspond to the following formula (I): 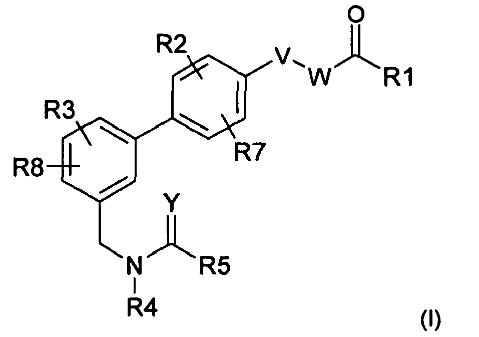 in which:
- **R1** represents a hydroxyl radical, an -OR6 radical or a hydroxylamine radical; R6 being defined below,
- **R2** and **R3,** which are identical or different, represent a hydrogen atom, a halogen atom, an alkyl radical comprising 1 to 12 carbon atoms, a hydroxyl radical, an alkoxy radical, a polyether radical, an aralkyl radical, an aryl radical or an amino radical which can be substituted by one or two identical or different radicals chosen from an alkyl radical comprising 1 to 12 carbon atoms or an aralkyl radical;
- **R4** represents a hydrogen atom or a lower alkyl radical having from 1 to 4 carbon atoms;
- **R5** represents an alkyl radical having from 1 to 12 carbon atoms, an aryl radical, an aralkyl radical, a heteroaryl radical, a heterocyclic radical or a 9-fluorenylmethyl radical;
- **R6** represents an alkyl, aryl or aralkyl radical;
- **R7** and **R8,** which are identical or different, represent a hydrogen atom, a halogen atom, an alkyl radical comprising 1 to 12 carbon atoms, a hydroxyl radical, an alkoxy radical, a polyether radical, an aralkyl radical, an aryl radical or an amino radical which can be substituted by one or two identical or different radicals chosen from an alkyl radical comprising 1 to 12 carbon atoms or an aralkyl radical;
- **Y** represents an oxygen or sulphur atom;
- **V-W** represents a CH₂-CH₂ or CH = CH sequence,
wherein:
- "alkyl radical of 1 to 12 carbon atoms" means a linear, branched or cyclic, saturated or unsaturated carbon-based chain that may be interrupted with a hetero atom and that may be substituted with one or more radicals chosen from a halogen atom, a hydroxyl radical, an alkoxy radical and a heterocyclic radical.
- "aryl radical" means a phenyl, biphenyl, cinnamyl or naphthyl radical that may be substituted with a halogen atom, a CF3 radical, an alkyl radical of 1 to 12 carbon atoms, an alkoxy radical, a nitro function, a polyether radical, an aryl radical, a benzoyl radical, an alkyl ester group, a carboxylic acid, a hydroxyl radical optionally protected with an acetyl or benzoyl group or an amino radical optionally protected with an acetyl or benzoyl group or optionally substituted with at least one alkyl containing from 1 to 12 carbon atoms, an aralkoxy radical, a phenoxy radical or an amide radical H2NCO.
- "aralkyl radical" means an alkyl radical of 1 to 12 carbon atoms substituted with an aryl radical or with a heteroaryl radical.
- "alkoxy radical" means an oxygen atom substituted with an alkyl radical of 1 to 12 carbon atoms
- "aralkoxy radical" means an oxygen atom substituted with an aralkyl radical.
- "polyether radical" means a radical containing from 1 to 7 carbon atoms interrupted with at least one oxygen atoms.
- "heteroaryl radical" means an aryl radical interrupted with one or more hetero atoms, optionally substituted with at least one halogen, an alkyl radical containing from 1 to 12 carbon atoms, an alkoxy radical, an aryl radical, a nitro function, a polyether radical, a heteroaryl radical, a benzoyl radical, an alkyl ester group, a carboxylic acid, a hydroxyl optionally protected with an acetyl or benzoyl group or an amino radical optionally protected with an acetyl or benzoyl group or optionally substituted with at least one alkyl containing from 1 to 12 carbon atoms,
and the salts of the compounds of formula (I).

2. Compounds according to Claim 1, **characterized in that** they are provided in the form of salts of an alkali metal or alkaline earth metal or of salts of an organic amine.

3. Compounds according to Claim 1, **characterized in that** they are provided, when they have an amine functional group, in the form of salts of an inorganic acid or of salts of an organic acid.

4. Compounds according to one of Claims 1 to 3, **characterized in that** the alkyl radicals having from 1 to 12 carbon atoms are chosen from the methyl, ethyl, propyl, isopropyl, butyl, tert-butyl, isoamyl, amyl, hexyl, heptyl, octyl, decyl, cyclohexyl, methylcyclohexyl, methylcyclobutyl, methylcyclopentyl or methylcyclohexyl radicals.

5. Compounds according to any one of Claims 1 to 4, **characterized in that** the lower alkyl radicals having from 1 to 4 carbon atoms are chosen from the methyl, ethyl, propyl, methylcyclopropyl, isopropyl, tert-butyl or n-butyl radicals.

6. Compounds according to any one of Claims 1 to 5, **characterized in that** the aryl radicals are chosen from the phenyl, biphenyl, cinnamyl or naphthyl radicals which can be substituted by a halogen atom, a CF₃ radical, an alkyl radical comprising 1 to 12 carbon atoms, an alkoxy radical, a nitro functional group, a polyether radical, an aryl radical, a benzoyl radical, an alkyl ester group, a carboxylic acid, a hydroxyl radical optionally protected by an acetyl or benzoyl group or an amino radical optionally protected by an acetyl or benzoyl group or optionally substituted by at least one alkyl having from 1 to 12 carbon atoms, an aralkoxy radical, a phenoxy radical or an amide radical H₂NCO.

7. Compounds according to any one of Claims 1 to 6, **characterized in that** the aralkyl radicals are alkyl radicals comprising 1 to 12 carbon atoms which are substituted by an aryl radical or by a heteroaryl radical.

8. Compounds according to any one of Claims 1 to 7, **characterized in that** the halogen atom is chosen from fluorine, chlorine, bromine or iodine atoms.

9. Compounds according to any one of Claims 1 to 8, **characterized in that** the alkoxy radicals are chosen from the methoxy, ethoxy, isopropyloxy, n-propyloxy, tert-butoxy, n-butoxy, n-pentyloxy, n-hexyloxy, cyclopropylmethoxy, cyclobutylmethoxy, cyclopentylmethoxy or cyclohexylmethoxy radicals.

10. Compounds according to any one of Claims 1 to 9, **characterized in that** the polyether radicals are chosen from radicals having from 1 to 7 carbon atoms which are interrupted by at least one oxygen atom and preferably the radicals such as the methoxyethoxy, ethoxyethoxy or methoxyethoxyethoxy radicals.

11. Compounds according to any one of Claims 1 to 10, **characterized in that** the heteroaryl radical is chosen from the group consisting of a pyridyl, furyl, thienyl, isoxazolyl, oxadiazolyl, oxazolyl, isothiazolyl, quinozalinyl, benzothiadiazolyl, benzimidazolyl, indolyl and benzofuranyl radical which are optionally substituted by at least one halogen, one alkyl radical having from 1 to 12 carbon atoms, one alkoxy radical, one aryl radical, one nitro functional group, one polyether radical, one heteroaryl radical, one benzoyl radical, one alkyl ester group, one carboxylic acid, one hydroxyl optionally protected by an acetyl or benzoyl group or one amino radical optionally protected by an acetyl or benzoyl group or optionally substituted by at least one alkyl having from 1 to 12 carbon atoms.

12. Compounds according to any one of Claims 1 to 11, **characterized in that** the heterocyclic radical is chosen from a morpholino, pyrrolidino, piperidino, piperazino, 2-oxopiperidin-1-yl and 2-oxopyrrolidin-1-yl radical which are optionally substituted by at least one alkyl radical having from 1 to 12 carbon atoms, one alkoxy radical, one aryl radical, one nitro functional group, one polyether radical, one heteroaryl radical, one benzoyl radical, one alkyl ester group, one carboxylic acid, one hydroxyl optionally protected by an acetyl or benzoyl group or one amino radical optionally protected by an acetyl or benzoyl group or optionally substituted by at least one alkyl having from 1 to 12 carbon atoms.

13. Compounds according to Claim 1, **characterized in that** they are taken, alone or as mixtures, from the group consisting of:
- 3-{3'-[((Methyl)(octanoyl)amino)methyl]biphenyl-4-yl}acrylic acid
- 3-{3'-[((Methyl)(octanoyl)amino)methyl]biphenyl-4-yl}propanoic acid
- 3-{3-Fluoro-3'-[((methyl)(octanoyl)amino)methyl]biphenyl-4-yl}acrylic acid
- 3-{3-Fluoro-3'-[((methyl)(octanoyl)amino)methyl]biphenyl-4-yl}propanoic acid
- 3-[3'-((Octanoylamino)methyl)biphenyl-4-yl]acrylic acid
- 3-[3'-((Octanoylamino)methyl)biphenyl-4-yl]propanoic acid
- 3-{3-Hydroxy-3'-[((methyl)(octanoyl)amino)methyl]biphenyl-4-yl}propanoic acid
- 3-{2-Butoxy-3'-[((methyl)(octanoyl)amino)methyl]biphenyl-4-yl}propanoic acid
- 3-{2-Butoxy-3'-[((hexanoyl)(methyl)amino)methyl]biphenyl-4-yl}propanoic acid
- 3-{2-Butoxy-3'-[((methyl)(pentanoyl)amino)methyl]biphenyl-4-yl}propanoic acid
- 3-{2-Butoxy-3'-[((heptanoyl)(methyl)amino)methyl]biphenyl-4-yl}propanoic acid
- 3-(2-Butoxy-3'-{[(4-ethoxybenzoyl)(methyl)amino]-methyl}biphenyl-4-yl)propanoic acid
- 3-(2-Butoxy-3'-{[(4-butoxybenzoyl)(methyl)amino]-methyl}biphenyl-4-yl)propanoic acid
- 3-{3'-[((Benzoyl)(methyl)amino)methyl]-2-butoxy-biphenyl-4-yl}propanoic acid
- 3-(2-Butoxy-3'-{[(4-methoxybenzoyl)(methyl)amino]-methyl}biphenyl-4-yl)propanoic acid
- 3-(2-Butoxy-3'-{[(3-methoxybenzoyl)(methyl)amino]-methyl}biphenyl-4-yl)propanoic acid
- (E)-3-{2-Fluoro-3'-[((methyl)(octanoyl)amino)methyl]biphenyl-4-yl}acrylic acid
- 3-{2-Fluoro-3'-[((methyl)(octanoyl)amino)methyl]biphenyl-4-yl}propanoic acid
- 3-{2-(2-Methoxyethoxy)-3'-[((methyl)(octanoyl)amino)methyl]biphenyl-4-yl}propanoic acid
- 3-{2-(3-Methylbutoxy)-3'-[((methyl)(octanoyl)amino)methyl]biphenyl-4-yl}propanoic acid
- 3-{2-(3-Chloropropoxy)-3'-[((methyl)(octanoyl)amino)methyl]biphenyl-4-yl}propanoic acid
- 3-{2-Methoxy-3'-[((methyl)(octanoyl)amino)methyl]biphenyl-4-yl}propanoic acid
- 3-{2-Methyl-3'-[((methyl)(octanoyl)amino)methyl]biphenyl-4-yl}propanoic acid
- 3-(3'-{[Methyl(4-phenylbutyryl)amino]methyl}-biphenyl-4-yl)propanoic acid
- (E)-3-{2'-Methyl-5'-[((methyl)(octanoyl)amino)methyl]biphenyl-4-yl}acrylic acid
- 3-{2'-Methyl-5'-[((methyl)(octanoyl)amino)methyl]biphenyl-4-yl}propanoic acid
- (E)-3-{2-Benzyloxy-3'-[((methyl)(octanoyl)amino)methyl]biphenyl-4-yl}acrylic acid
- 3-{2-Benzyloxy-3'-[((methyl)(octanoyl)amino)methyl]biphenyl-4-yl}propanoic acid
- 3-{3'-[((Methyl)(octanoyl)amino)methyl]-2-propoxy-biphenyl-4-yl}propanoic acid
- (E)-3-{3,5-Difluoro-3'-[((methyl)(octanoyl)amino)methyl]biphenyl-4-yl}acrylic acid
- 3-{3,5-Difluoro-3'-[((methyl)(octanoyl)amino)methyl]biphenyl-4-yl}propanoic acid
- 3-(3'-{[(4-Butoxybenzoyl)(methyl)amino]methyl}-biphenyl-4-yl)propanoic acid
- 3-(3'-{[(4-Butoxybenzoyl)(ethyl)amino]methyl}-biphenyl-4-yl)propanoic acid
- 3-{2-Cyclopropylmethoxy-3'-[((methyl)(octanoyl)amino)methyl]biphenyl-4-yl}propanoic acid
- 3-{2-Ethoxy-3'-[((methyl)(octanoyl)amino)methyl]biphenyl-4-yl}propanoic acid
- 3-[3'-[((Methyl)(octanoyl)amino)methyl]-2-(3,3,3-trifluoropropoxy)biphenyl-4-yl]propanoic acid
- 3-[3'-[((Methyl)(octanoyl)amino)methyl]-2-(4,4,4-trifluorobutoxy)biphenyl-4-yl]propanoic acid
- 3-{2-(3-Hydroxypropoxy)-3'-[((methyl)(octanoyl)amino)methyl]biphenyl-4-yl}propanoic acid
- 3-{2-(4-Hydroxybutoxy)-3'-[((methyl)(octanoyl)amino)methyl]biphenyl-4-yl}propanoic acid
- 3-{2-(3-Fluorobenzyloxy)-3'-[((methyl)(octanoyl)amino)methyl]biphenyl-4-yl}propanoic acid
- 3-{2-(4-Fluorobenzyloxy)-3'-[((methyl)(octanoyl)amino)methyl]biphenyl-4-yl}propanoic acid
- 3-{3'-[((Methyl)(octanoyl)amino)methyl]-2-(pentyloxy)biphenyl-4-yl}propanoic acid
- 3-{3'-[((Hexanoyl)(methyl)amino)methyl]-2-(pentyloxy)biphenyl-4-yl}propanoic acid
- 3-{2-(2-Ethoxyethoxy)-3'-[((hexanoyl)(methyl)amino)methyl]biphenyl-4-yl}propanoic acid
- 3-{2-(2-(Diethylamino)ethoxy)-3'-[((methyl)(octanoyl)amino)methyl]biphenyl-4-yl}propanoic acid
- 3-[3'-[((Methyl)(octanoyl)amino)methyl]-2-(2-(morpholin-4-yl)ethoxy)biphenyl-4-yl]propanoic acid
- 3-{2-Amino-3'-[((hexanoyl)(methyl)amino)methyl]biphenyl-4-yl}propanoic acid
- 3-{2-Butylamino-3'-[((hexanoyl)(methyl)amino)methyl]biphenyl-4-yl}propanoic acid
- 3-{2-Benzylamino-3'-[((hexanoyl)(methyl)amino)methyl]biphenyl-4-yl}propanoic acid
- 3-{2-Diethylamino-3'-[((hexanoyl)(methyl)amino)methyl]biphenyl-4-yl}propanoic acid
- 3-{3'-[((Hexanoyl)(methyl)amino)methyl]-2-(propylamino)biphenyl-4-yl}propanoic acid
- 3-{2-(4-Fluorobenzylamino)-3'-[((hexanoyl)(methyl)amino)methyl]biphenyl-4-yl}propanoic acid
- 3-{2-Butylamino-3'-[((methyl)(octanoyl)amino)methyl]biphenyl-4-yl}propanoic acid
- 3-{2-Benzylamino-3'-[((methyl)(octanoyl)amino)methyl]biphenyl-4-yl}propanoic acid
- 3-{2-Diethylamino-3'-[((methyl)(octanoyl)amino)methyl]biphenyl-4-yl}propanoic acid
- 3-{3'-[((Methyl)(octanoyl)amino)methyl]-2-(propylamino)biphenyl-4-yl}propanoic acid
- 3-{2-(4-Fluorobenzylamino)-3'-[((methyl)(octanoyl)amino)methyl]biphenyl-4-yl}propanoic acid
- 3-{2-Cyclohexylmethoxy-3'-[((methyl)(octanoyl)amino)methyl]biphenyl-4-yl}propanoic acid
- 3-{2-Cyclopentylmethoxy-3'-[((methyl)(octanoyl)amino)methyl]biphenyl-4-yl}propanoic acid
- N-[2'-(2-Cyclobutylethoxy)-4'-(2-(hydroxycarbamoyl)ethyl)biphenyl-3-ylmethyl](methyl)octanamide
- 3-[3'-[((Methyl)(octanoyl)amino)methyl]-2-(3-(trifluoromethyl)benzyloxy)biphenyl-4-yl]propanoic acid
- 3-[3'-[((Hexanoyl)(methyl)amino)methyl]-2-(4-(trifluoromethyl)benzyloxy)biphenyl-4-yl]propanoic acid
- 3-{2-(3-Carbamoylbenzyloxy)-3'-[((methyl)(octanoyl)amino)methyl]biphenyl-4-yl}propanoic acid
- 3-[3'-[((Methyl)(octanoyl)amino)methyl]-2-(2-(piperazin-1-yl)ethoxy)biphenyl-4-yl]propanoic acid
- 3-[3'-[((Methyl)(octanoyl)amino)methyl]-2-(2-(pyrrolidin-1-yl)ethoxy)biphenyl-4-yl]propanoic acid
- 3-[2-(3-Methoxybenzyloxy)-3'-({[3-(3-methoxyphenyl)propionyl](methyl)amino}methyl)biphenyl-4-yl]propanoic acid
- 3-[2-(4-(tert-Butyl)benzyloxy)-3'-({methyl[3-(3-phenoxyphenyl)propionyl]amino}methyl)biphenyl-4-yl]propanoic acid
- 3-(2-(3,5-Dimethoxybenzyloxy)-3'-{[methyl(3-phenoxybenzoyl)amino]methyl}biphenyl-4-yl)propanoic acid
- 3-[3'-{[Methyl(4-phenoxybenzoyl)amino]methyl}-2-(3-(trifluoromethyl)benzyloxy)biphenyl-4-yl]propanoic acid
- 3-[2-(3-Isopropoxybenzyloxy)-3'-({[3-(4-methoxyphenyl)propionyl](methyl)amino}methyl)biphenyl-4-yl]propanoic acid
- 3-[2'-(3-Methoxybenzyloxy)-5'-({[3-(3-methoxyphenyl)propionyl](methyl)amino}methyl)biphenyl-4-yl]propanoic acid
- 3-{2'-Cyclohexylmethoxy-5'-[((hexanoyl)(methyl)amino)methyl]biphenyl-4-yl}propanoic acid
- 3-{4'-Ethoxy-3'-[((hexanoyl)(methyl)amino)methyl]-2-propoxybiphenyl-4-yl}propanoic acid
- 3-{3'-[((Hexanoyl)(methyl)amino)methyl]-3,5-dimethoxybiphenyl-4-yl}propanoic acid
- 3-[3,5-Diethoxy-3'-({[3-(3-methoxyphenyl)-propionyl](methyl)amino}methyl)biphenyl-4-yl]propanoic acid
- 3-[3'-({[3-(4-Methoxyphenyl)propionyl](methyl)amino}methyl)-3-propoxybiphenyl-4-yl]propanoic acid
- 3-{3-Cyclopropylmethoxy-3'-[((hexanoyl)(methyl)amino)methyl]biphenyl-4-yl}propanoic acid
- 3-{3-Ethoxy-4'-fluoro-3'-[((hexanoyl)(methyl)amino)methyl]biphenyl-4-yl}propanoic acid
- 3-[3'-[((Hexanoyl)(methyl)amino)methyl]-3-(4,4,4-trifluorobutoxy)biphenyl-4-yl]propanoic acid
- 3-{3-Benzyloxy-3'-[((hexanoyl)(methyl)amino)methyl]biphenyl-4-yl}propanoic acid
- 3-[3'-({[3-(4-Methoxyphenyl)propionyl](methyl)amino}methyl)-3-(3-(trifluoromethyl)benzyloxy)biphenyl-4-yl]propanoic acid
- 3-[3'-({[3-(3-Methoxyphenyl)propionyl](methyl)amino}methyl)-3,5-dipropylbiphenyl-4-yl]propanoic acid
- 3-{3-(2,2-Dimethylpropyl)-3'-[((hexanoyl)(methyl)amino)methyl]biphenyl-4-yl}propanoic acid
- 3-{3'-[((Hexanoyl)(methyl)amino)methyl]-3,5-dimethylbiphenyl-4-yl}propanoic acid
- 3-{3-[((Hexanoyl)(methyl)amino)methyl]-4"-methoxy-1,1';3',1"-terphenyl-4'-yl}propanoic acid
- 3-{3-[((Hexanoyl)(methyl)amino)methyl]-3"-methoxy-1,1';2',1"-terphenyl-4'-yl}propanoic acid
- 3-[3-({[3-(3-Methoxyphenyl)propionyl](methyl)amino}methyl)-3"-trifluoromethyl-1,1';2',1"-terphenyl-4'-yl]propanoic acid
- 3-{3'-[((Hexanoyl)(methyl)amino)methyl]-2-[2-(3-isopropoxyphenyl)ethyl]biphenyl-4-yl}propanoic acid
- 3-{3'-[((Hexanoyl)(methyl)amino)methyl]-2-[(pyridin-3-ylmethyl)amino]biphenyl-4-yl}propanoic acid
- 3-[3'-[((Hexanoyl)(methyl)amino)methyl]-3-(2-methoxyethylamino)biphenyl-4-yl]propanoic acid
- Methyl 3-{3,5-diethyl-3'-[((hexanoyl)(methyl)amino)methyl]biphenyl-4-yl}propanoate
- Methyl 3-[3'-{[methyl(3-phenoxybenzoyl)amino]-methyl}-2-(3-(trifluoromethyl)benzyloxy)biphenyl-4-yl]propanoate
- Methyl 3-[3'-{[(3-(biphenyl-4-yl)propionyl)(methyl)amino]methyl}-2-(3-methoxybenzyloxy)biphenyl-4-yl]propanoate
- Ethyl 3-[3'-({[3-(3-methoxyphenyl)propionyl](methyl)amino}methyl)-2-(4,4,4-trifluorobutoxy)biphenyl-4-yl]propanoate
- N-[4'-(2-(Hydroxycarbamoyl)ethyl)-4"-methoxy-1,1';3',1"-terphenyl-3-ylmethyl](methyl)hexanamide.

14. Compounds according to Claim 1 or 2, **characterized in that** they exhibit one at least of the following characteristics:
- **R1** represents a hydroxyl radical;
- **R2** and **R7** represent an alkoxy or aryloxy radical, an alkylamino radical or a polyether radical;
- **R3** and **R8** represent a hydrogen atom;
- **R4** represents a lower alkyl radical comprising 1 to 4 carbon atoms;
- **R5** represents an alkyl radical comprising 3 to 8 carbon atoms or an aryl radical;
- **Y** is an oxygen atom;
- the **V-W** bond is a CH2-CH2 or CH=CH sequence.

15. Compounds according to Claim 14, **characterized in that** they exhibit all of the following characteristics:
- **R1** represents a hydroxyl radical;
- **R2** and **R7** represent an alkoxy or aryloxy radical, an alkylamino radical or a polyether radical;
- **R3** and **R8** represent a hydrogen atom;
- **R4** represents a lower alkyl radical comprising 1 to 4 carbon atoms;
- **R5** represents an alkyl radical comprising 3 to 8 carbon atoms or an aryl radical;
- **Y** is an oxygen atom;
- the **V-W** bond is a CH2-CH2 or CH=CH sequence.

16. Cosmetic composition, **characterized in that** it comprises, in a physiologically acceptable vehicle, at least one of the compounds as defined in any one of Claims 1 to 15.

17. Composition according to Claim 16, **characterized in that** the concentration of compound(s) according to one of Claims 1 to 15 is between 0.001% and 3% by weight, with respect to the total weight of the composition.

18. Cosmetic use of a composition as defined in either of Claims 16 and 17 for body or hair hygiene.

19. Compounds according to any one of Claims 1 to 15, as medicament.

20. Use of a compound according to any one of Claims 1 to 15 in the manufacture of a composition intended to regulate and/or restore the metabolism of skin lipids.

21. Use of a compound according to any one of Claims 1 to 15 in the manufacture of a composition intended for the treatment:
- of dermatological conditions linked to a disorder of keratinization involving differentiation and proliferation, in particular acne vulgaris, comedonic or polymorphic acne, acne rosacea, nodulocystic acne, acne conglobata, senile acne or secondary acnes, such as solar, drug or occupational acne,
- of ichthyosis, of ichthyosiform conditions, of Darier's disease, of palmoplantar keratoderma, of leucoplakia and leucoplakiform conditions, or of cutaneous or mucosal (oral) lichen,
- of dermatological conditions with an inflammatory immunoallergic component, with or without cell proliferation disorder, in particular cutaneous, mucosal or ungual psoriasis, psoriatic rheumatism, cutaneous atopy, such as eczema, respiratory atopy or gingival hypertrophy,
- of benign or malignant dermal or epidermal proliferations of viral or nonviral origin, in particular common warts, flat warts, epidermodysplasia verruciformis, florid or oral papillomatoses, or T lymphoma,
- of proliferations which can be induced by ultraviolet radiation, in particular basal cell and prickle cell epithelioma,
- of precancerous skin lesions, in particular keratoacanthomas,
- of immune dermatoses, in particular lupus erythematosus,
- of immune bullous diseases,
- of collagen diseases, in particular scleroderma,
- of dermatological or general conditions with an immunological component,
- of skin disorders due to exposure to UV radiation, of photoinduced or chronologic skin ageing or of actinic keratoses and pigmentations, or any pathology associated with chronologic or actinic ageing, in particular xerosis,
- of disorders of the sebaceous function, in particular hyperseborrhoea of acne or simple seborrhoea,
- of disorders of cicatrization or of stretch marks,
- of disorders of pigmentation, such as hyperpigmentation, melasma, hypopigmentation or vitiligo,
- of conditions of the metabolism of lipids, such as obesity, hyperlipidaemia or non-insulin-dependent diabetes,
- of inflammatory conditions, such as arthritis,
- of cancerous or precancerous conditions,
- of alopecia of various origins, in particular alopecia due to chemotherapy or to radiation,
- of disorders of the immune system, such as asthma, type I diabetes mellitus, multiple sclerosis or other selective dysfunctions of the immune system,
- of conditions of the cardiovascular system, such as arteriosclerosis or hypertension.

22. Pharmaceutical composition, **characterized in that** it comprises, in a physiologically acceptable vehicle, at least one of the compounds as defined in any one of Claims 1 to 15.

23. Composition according to Claim 22, **characterized in that** the concentration of compound(s) according to one of Claims 1 to 15 is between 0.001% and 10% by weight, with respect to the total weight of the composition.

24. Composition according to Claim 22, **characterized in that** the concentration of compound(s) according to one of Claims 1 to 15 is between 0.01% and 1% by weight, with respect to the total weight of the composition.

## Patentansprüche

1. Verbindungen, **dadurch gekennzeichnet, dass** sie der nachstehenden Formel (I) entsprechen: 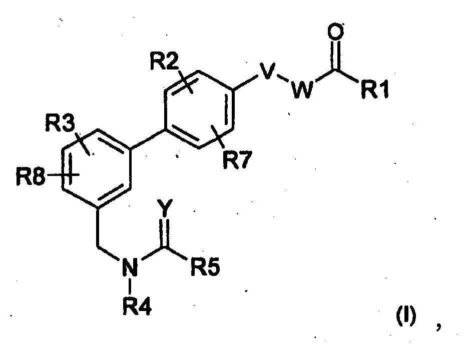 worin bedeuten:
- **R1** eine Hydroxylgruppe, eine Gruppe -OR6 oder eine Hydroxylamingruppe, wobei R6 wie unten definiert ist,
- **R2** und **R3,** die gleich oder verschieden sind, ein Wasserstoffatom, ein Halogenatom, eine Alkylgruppe mit 1 bis 12 Kohlenstoffatomen, eine Hydroxylgruppe, eine Alkoxygruppe, eine Polyethergruppe, eine Aralkylgruppe, eine Arylgruppe oder eine Aminogruppe, die mit einer oder zwei identischen oder unterschiedlichen Gruppen substituiert sein kann, die unter Alkylgruppen mit 1 bis 12 Kohlenstoffatomen oder Aralkylgruppen ausgewählt sind;
- **R4** ein Wasserstoffatom oder eine niedere Alkylgruppe mit 1 bis 4 Kohlenstoffatomen;
- **R5** eine Alkylgruppe mit 1 bis 12 Kohlenstoffatomen, eine Arylgruppe, eine Aralkylgruppe, eine Heteroarylgruppe, eine heterocyclische Gruppe oder eine 9-Fluorenylmethyl-Gruppe;
- **R6** eine Alkylgruppe, eine Arylgruppe oder eine Aralkylgruppe;
- **R7** und **R8,** die gleich oder verschieden sind, ein Wasserstoffatom, ein Halogenatom, eine Alkylgruppe mit 1 bis 12 Kohlenstoffatomen, eine Hydroxylgruppe, eine Alkoxygruppe, eine Polyethergruppe, eine Aralkylgruppe, eine Arylgruppe oder eine Aminogruppe, die mit einer oder zwei identischen oder verschiedenen Gruppen substituiert sein kann, die unter Alkylgruppen mit 1 bis 12 Kohlenstoffatomen oder einer Aralkylgruppe ausgewählt sind;
- **Y** ein Sauerstoffatom oder ein Schwefelatom;
- **V-W** eine Gruppierung CH₂-CH₂ oder CH=CH,
wobei
- "Alkylgruppe mit 1 bis 12 Kohlenstoffatomen" eine geradkettige, verzweigte oder cyclische, gesättigte oder ungesättigte Kette auf Kohlenstoffbasis bedeutet, die durch ein Heteroatom unterbrochen und durch einen oder mehrere Substituenten substituiert sein kann, die unter einem Halogenatom, einer Hydroxylgruppe, einer Alkoxygruppe und einer heterocyclischen Gruppe ausgewählt sind,
- "Arylgruppe" eine Phenylgruppe, eine Biphenylgruppe, eine Cinnamylgruppe oder eine Naphthylgruppe bedeutet, die substituiert sein kann mit einem Halogenatom, einer Gruppe CF₃, einer Alkylgruppe mit 1 bis 12 Kohlenstoffatomen, einer Alkoxygruppe, einer Nitrofunktion, einer Polyethergruppe, einer Arylgruppe, einer Benzoylgruppe, einer Alkylestergruppe, einer Carbonsäuregruppe, einer Hydroxylgruppe, die gegebenenfalls mit einer Acetylgruppe oder einer Benzoylgruppe geschützt ist, oder einer Aminogruppe, die gegebenenfalls mit einer Acetylgruppe oder einer Benzoylgruppe geschützt oder gegebenenfalls mit mindestens einer Alkylgruppe mit 1 bis 12 Kohlenstoffatomen, einer Aralkoxygruppe, einer Phenoxygruppe oder einer Amidgruppe H₂NCO substituiert ist,
- "Aralkylgruppe" eine Alkylgruppe mit 1 bis 12 Kohlenstoffatomen bedeutet, die mit einer Arylgruppe oder einer Heteroarylgruppe substituiert ist,
- "Alkoxygruppe" ein Sauerstoffatom bedeutet, das mit einer Alkylgruppe mit 1 bis 12 Kohlenstoffatomen substituiert ist,
- "Aralkoxygruppe" ein Sauerstoffatom bedeutet, das mit einer Aralkylgruppe substituiert ist,
- "Polyethergruppe" eine Gruppe mit 1 bis 7 Kohlenstoffatomen bedeutet, die durch mindestens ein Sauerstoffatom unterbrochen ist,
- "Heteroarylgruppe" eine Arylgruppe bedeutet, die durch ein oder mehrere Heteroatome unterbrochen ist und wahlweise substituiert ist mit mindestens einem Halogen, einer Alkylgruppe mit 1 bis 12 Kohlenstoffatomen, einer Alkoxygruppe, einer Arylgruppe, einer Nitrofunktion, einer Polyethergruppe, einer Heteroarylgruppe, einer Benzoylgruppe, einer Alkylestergruppe, einer Carbonsäuregruppe, einer Hydroxylgruppe, die gegebenenfalls mit einer Acetylgruppe oder eine Benzoylgruppe geschützt ist, oder einer Aminogruppe, die gegebenenfalls mit einer Acetylgruppe oder einer Benzoylgruppe geschützt ist oder wahlweise mit mindestens einer Alkylgruppe mit 1 bis 12 Kohlenstoffatomen substituiert ist,
sowie die Salze der Verbindungen der Formel (I).

2. Verbindungen nach Anspruch 1, **dadurch gekennzeichnet, dass** sie in Form von Salzen eines Alkalimetalls oder eines Erdalkalimetalls oder in Form von Salzen eines organischen Amins vorliegen.

3. Verbindungen nach Anspruch 1, **dadurch gekennzeichnet, dass** sie, wenn sie eine funktionelle Aminogruppe aufweisen, in Form von Salzen einer anorganischen Säure oder von Salzen einer organischen Säure vorliegen.

4. Verbindungen nach einem der Ansprüche 1 bis 3, **dadurch gekennzeichnet, dass** die Alkylgruppen mit 1 bis 12 Kohlenstoffatomen ausgewählt sind unter den Gruppen Methyl, Ethyl, Propyl, Isopropyl, Butyl, tert.-Butyl, Isoamyl, Amyl, Hexyl, Heptyl, Octyl, Decyl, Cyclohexyl, Methylcyclohexyl, Methylcyclobutyl, Methylcyclopentyl oder Methylcyclohexyl.

5. Verbindungen nach einem der Ansprüche 1 bis 4, **dadurch gekennzeichnet, dass** die niederen Alkylgruppen mit 1 bis 4 Kohlenstoffatomen ausgewählt sind unter den Gruppen Methyl, Ethyl, Propyl, Methylcyclopropyl, Isopropyl, tert.-Butyl oder n-Butyl.

6. Verbindungen nach einem der Ansprüche 1 bis 5, **dadurch gekennzeichnet, dass** die Arylgruppen ausgewählt sind unter den Gruppen Phenyl, Biphenyl, Cinnamyl oder Naphthyl, die substituiert sein können mit einem Halogenatom, einer Gruppe CF₃, einer Alkylgruppe mit 1 bis 12 Kohlenstoffatomen, einer Alkoxygruppe, einer funktionellen Nitrogruppe, einer Polyethergruppe, einer Arylgruppe, einer Benzoylgruppe, einer Alkylestergruppe, einer Carbonsäuregruppe, einer Hydroxylgruppe, die gegebenenfalls durch eine Acetylgruppe oder eine Benzoylgruppe geschützt ist, oder einer Aminogruppe, die gegebenenfalls durch eine Acetylgruppe oder eine Benzoylgruppe geschützt oder wahlweise mit mindestens einer Alkylgruppe mit 1 bis 12 Kohlenstoffatomen, einer Aralkoxygruppe, einer Phenoxygruppe oder einer Amidgruppe H₂NCO substituiert ist.

7. Verbindungen nach einem der Ansprüche 1 bis 6, **dadurch gekennzeichnet, dass** die Aralkylgruppen Alkylgruppen mit 1 bis 12 Kohlenstoffatomen sind, die mit einer Arylgruppe oder einer Heteroarylgruppe substituiert sind.

8. Verbindungen nach einem der Ansprüche 1 bis 7, **dadurch gekennzeichnet, dass** das Halogenatom unter Fluor, Chlor, Brom oder Iod ausgewählt ist.

9. Verbindungen nach einem der Ansprüche 1 bis 8, **dadurch gekennzeichnet, dass** die Alkoxygruppen ausgewählt sind unter den Gruppen Methoxy, Ethoxy, Isopropyloxy, n-Propyloxy, tert.-Butoxy, n-Butoxy, n-Pentyloxy, n-Hexyloxy, Cyclopropylmethoxy, Cyclobutylmethoxy, Cyclopentylmethoxy oder Cyclohexylmethoxy.

10. Verbindungen nach einem der Ansprüche 1 bis 9, **dadurch gekennzeichnet, dass** die Polyethergruppen ausgewählt sind unter Gruppen mit 1 bis 7 Kohlenstoffatomen, die durch mindestens ein Sauerstoffatom unterbrochen sind, und bevorzugt Gruppen wie Methoxyethoxy, Ethoxyethoxy oder Methoxyethoxyethoxy.

11. Verbindungen nach einem der Ansprüche 1 bis 10, **dadurch gekennzeichnet, dass** die Heteroarylgruppe aus der Gruppe ausgewählt ist, die besteht aus Pyridyl, Furyl, Thienyl, Isoxazyl, Oxadiazolyl, Oxazolyl, Isothiazolyl, Chinozalinyl, Benzothiadiazolyl, Benzimidazolyl, Indolyl und Benzofuranyl, die gegebenenfalls substituiert sind mit mindestens einem Halogen, einer Alkylgruppe mit 1 bis 12 Kohlenstoffatomen, einer Alkoxygruppe, einer Arylgruppe, einer funktionellen Nitrogruppe, einer Polyethergruppe, einer Heteroarylgruppe, einer Benzoylgruppe, einer Alkylestergruppe, einer Carbonsäuregruppe, einer Hydroxylgruppe, die wahlweise durch eine Acetylgruppe oder eine Benzoylgruppe geschützt ist, oder einer Aminogruppe, die wahlweise durch eine Acetylgruppe oder eine Benzoylgruppe geschützt oder wahlweise mit mindestens einer Alkylgruppe mit 1 bis 12 Kohlenstoffatomen substituiert ist.

12. Verbindungen nach einem der Ansprüche 1 bis 11, **dadurch gekennzeichnet, dass** die heterocyclische Gruppe ausgewählt ist unter den Gruppen Morpholino, Pyrrolidino, Piperidino, Piperazino, 2-Oxopiperidin-1-yl und 2-Oxopyrrolidin-1-yl, die gegebenenfalls substituiert sind mit mindestens einer Alkylgruppe mit 1 bis 12 Kohlenstoffatomen, einer Alkoxygruppe, einer Arylgruppe, einer funktionellen Nitrogruppe, einer Polyethergruppe, einer Heteroarylgruppe, einer Benzoylgruppe, einer Alkylestergruppe, einer Carboxylgruppe, einer Hydroxylgruppe, die wahlweise durch eine Acetylgruppe oder eine Benzoylgruppe geschützt ist, oder einer Aminogruppe, die wahlweise durch eine Acetylgruppe oder eine Benzoylgruppe geschützt oder gegebenenfalls mit mindestens einer Alkylgruppe mit 1 bis 12 Kohlenstoffatomen substituiert ist.

13. Verbindungen nach Anspruch 1, **dadurch gekennzeichnet, dass** sie allein oder in Form von Gemischen aus der Gruppe ausgewählt sind, die besteht aus:
- 3-{3'-[((Methyl)(octanoyl)amino)-methyl]-biphenyl-4-yl}-acrylsäure
- 3-{3'-[((Methyl)(octanoyl)amino)-methyl]-biphenyl-4-yl}-propansäure
- 3-{3-Fluor-3'-[((methyl)(octanoyl)amino)-methyl]-biphenyl-4-yl}-acrylsäure
- 3-{3-Fluor-3'-[((methyl)(octanoyl)amino)-methyl]-biphenyl-4-yl}-propansäure
- 3[3'-((Octanoylamino)methyl)-biphenyl-4-yl]-acrylsäure
- 3[3'-((Octanoylamino)methyl)-biphenyl-4-yl]-propansäure
- 3-{3-Hydroxy-3'-[((methyl)(octanoyl)amino)-methyl]-biphenyl-4-yl}-propansäure
- 3-{2-Butoxy-3'-[((methyl)(octanoyl)amino)-methyl]-biphenyl-4-yl}-propansäure
- 3-{2-Butoxy-3'-[((hexanoyl)(methyl)amino)-methyl]-biphenyl-4-yl}-propansäure
- 3-{2-Butoxy-3'-[((methyl)(pentanoyl)amino)-methyl]-biphenyl-4-yl}-propansäure
- 3-(2-Butoxy-3'-[((heptanoyl)(methyl)amino)-methyl}-biphenyl-4-yl)-propansäure
- 3-(2-Butoxy-3'-{[(4-butoxybenzoyl)(methyl)amino]-methyl}-biphenyl-4-yl)-propansäure
- 3-(2-Butoxy-3'-{[(4-butoxybenzoyl)(methyl)amino]-methyl}-biphenyl-4-yl)-propansäure
- 3-{3'-[((Benzoyl)(methyl)amino)-methyl]-2-butoxy-biphenyl-4-yl}-propansäure
- 3-(2-Butoxy-3'-{[(4-methoxybenzoyl)(methyl)amino]-methyl}-biphenyl-4-yl)-propansäure
- 3-(2-Butoxy-3'-{[(3-methoxybenzoyl)(methyl)amino]-methyl}-biphenyl-4-yl-propansäure
- (E)-3-{2-Fluor-3'-[((methyl)(octanoyl)amino)-methyl]-biphenyl-4-yl}-acrylsäure
- 3-{2-Fluor-3'-[((methyl)(octanoyl)amino)-methyl]-biphenyl-4-yl}-propansäure
- 3-{2-(2-Methoxyethoxy)-3'-[((methyl)(octanoyl)amino)-methyl]-biphenyl-4-yl}-propansäure
- 3-{2-(3-Methylbutoxy)-3'-[((methyl)(octanoyl)amino)-methyl]-biphenyl-4-yl}-propansäure
- 3-{2-(3-Chlorpropoxy}-3'-[((methyl)(octanoyl)amino)-methyl]-biphenyl-4-yl}-propansäure
- 3-{2-Methoxy-3'-[((methyl)(octanoyl)amino)-methyl]-biphenyl-4-yl}-propansäure
- 3-(2-Methyl-3'-[((methyl)(octanoyl)amino)-methyl]-biphenyl-4-yl}-propansäure
- 3-(3'-{[Methyl(4-phenylbutyryl)amino]-methyl)-biphenyl-4-yl)-propansäure
- (E)-3-{2'-Methyl-5'-[((methyl)(octanoyl)amino)-methyl]-biphenyl-4-yl-acrylsäure
- 3-{2'-Methyl-5'-[((methyl)(octanoyl)amino)-methyl]-biphenyl-4-yl}-propansäure
- (E)-3-{2-Benzyloxy-3'-[((methyl)(octanoyl)amino)-methyl]-biphenyl-4-yl}-acrylsäure
- 3-{2-Benzyloxy-3'-[((methyl)(octanoyl)amino)-methyl]-biphenyl-4-yl}-propansäure
- 3-{3'-[((Methyl)(octanoyl)amino)-methyl]-2-propoxy-biphenyl-4-yl}-propansäure
- (E)-3-{3,5-Difluor-3'-[((methyl)(octanoyl)amino)-methyl]-biphenyl-4-yl}-acrylsäure
- 3-{3,5-Difluor-3'-[((methyl)(octanoyl)amino)-methyl]-biphenyl-4-yl}-propansäure
- 3-(3'-{[(4-Butoxybenzoyl)(methyl)amino]-methyl}-biphenyl-4-yl}-propansäure
- 3-(3'-{[(4-Butoxybenzoyl)(ethyl)amino]-methyl}-biphenyl-4-yl)-propansäure
- 3-{2-Cyclopropylmethoxy-3'-[((methyl)(octanoyl)amino)-methyl]-biphenyl-4-yl}-propansäure
- 3-{2-Ethoxy-3'-[((methyl)(octanoyl)amino)-methyl]-biphenyl-4-yl}-propansäure
- 3-[3'-[((Methyl)(octanoyl)amino)-methyl]-2-(3,3,3-trifluorpropoxy)-biphenyl-4-yl]-propansäure
- 3-[3'-[((Methyl)(octanoyl]amino)-methyl]-2-(4,4,4-trifluorbutoxy)-biphenyl-4-yl]-propansäure
- 3-{2-(3-Hydroxypropoxy)-3'-[((methyl)(octanoyl)amino)-methyl]-biphenyl-4-yl}-propansäure
- 3-{2-(4-Hydroxybutoxy)-3'-[((methyl)(octanoyl)amino)-methyl]-biphenyl-4-yl}-propansäure
- 3-{2-(3-Fluorbenzyloxy)-3'-[((methyl)(octanoyl)amino)-methyl]-biphenyl-4-yl}-propansäure
- 3-{2-(4-Fluorbenzyloxy)-3'-[((methyl)(octanoyl)amino)-methyl]-biphenyl-4-yl}-propansäure
- 3-{3'-[((Methyl)(octanoyl)amino)-methyl]-2-(pentyloxy)-biphenyl-4-yl}-propansäure
- 3-{3'-[((Hexanoyl)(methyl)amino)-methyl)-2-(pentyloxy)-biphenyl-4-yl}-propansäure
- 3-{2-(2-Ethoxyethoxy)-3'-[((hexanoyl)(methyl)amino)-methyl]-biphenyl-4-yl}-propansäure
- 3-{2-(2-(Diethylamino)ethoxy)-3'-[((methyl)(octanoyl)amino)-methyl}-biphenyl-4-yl-propansäure
- 3-[3'-[((Methyl)(octanoyl)amino)-methyl]-2-(2-(morpholin-4-yl)ethoxy)-biphenyl-4-yl]-propansäure
- 3-{2-Amino-3'-[((hexanoyl)(methyl)amino)-methyl]-biphenyl-4-yl}-propansäure
- 3-{2-Butylamino-3'-[((hexanoyl)(methyl)amino)-methyl]-biphenyl-4-yl}-propansäure
- 3-{2-Benzylamino-3'-[((hexanoyl)(methyl)amino)-methyl]-biphenyl-4-yl}-propansäure
- 3-{2-Diethylamino-3'-[((hexanoyl)(methyl)amino)-methyl]-biphenyl-4-yl}-propansäure
- 3-{3'-((Hexanoyl)(methyl)amino)-methyl]-2-(propylamino)-biphenyl-4-yl-propansäure
- 3-{2-(4-Fluorbenzylamino)-3'-[((hexanoyl)(methyl)amino)-methyl]-biphenyl-4-yl-propansäure
- 3-{2-Butylamino-3'-[((methyl)(octanoyl)amino)-methyl]-biphenyl-4-yl-propansäure
- 3-{2-Benzylamino-3'-[((methyl)(octanoyl)amino)-methyl]-biphenyl-4-yl}-propansäure
- 3-{2-Diethylamino-3'-[((methyl)(octanoyl)amino)-methyl]-biphenyl-4-yl}-propansäure
- 3-{3'-[((Methyl)(octanoyl)amino)-methyl]-2-(propylamino)-biphenyl-4-yl}-propansäure
- 3-{2-(4-Fluorbenzylamino)-3'-[((methyl)(octanoyl)amino)-methyl]-biphenyl-4-yl-propansäure
- 3-{2-Cyclohexylmethoxy-3'-[((methyl)(octanoyl)amino)-methyl]-biphenyl-4-yl-propansäure
- 3-{2-Cyclopentylmethoxy-3-[((methyl)(octanoyl)amino)-methyl]-biphenyl-4-yl}-propansäure
- N-[2'-(2-Cyclobutylethoxy)-4'-(2-(hydroxycarbamoyl)-ethyl)-biphenyl-3-ylmethyl](methyl)-octanamid
- 3-[3'-[((Methyl)(octanoyl)amino)-methyl]-2-(3-(trifluormethyl)-benzyloxy)-biphenyl-4-yl-propansäure
- 3-[3'-[((Hexanoyl)(methyl)amino)-methyl]-2-(4-(trifluormethyl)-benzyloxy)-biphenyl-4-yl}-propansäure
- 3-{2-(3-Carbamoylbenzyloxy)-3'-[((methyl)(octanoyl)amino)-methyl]-biphenyl-4-yl-propansäure
- 3-[3'-[((Methyl)(octanoyl)amino)-methyl]-2-(2-(piperazin-1-yl)ethoxy)-biphenyl-4-yl]-propansäure
- 3-[3'-[((Methyl)(octanoyl)amino)-methyl]-2-(2-(pyrrolidin-1-yl)ethoxy)-biphenyl-4-yl]-propansäure
- 3-[2-(3-Methoxybenzyloxy)-3'-({[3-(3-methoxyphenyl)-propionyl]-(methyl)amino}-methyl)-biphenyl-4-yl]-propansäure
- 3-[2-(4-(tert.-Butyl)benzyloxy)-3'-({methyl[3-(3-phenoxyphenyl)-propionyl]amino}-methyl)-biphenyl-4-yl]-propansäure
- 3-(2-(3,5-Dimethoxybenzyloxy)-3'-{[methyl(3-phenoxybenzoyl)-amino]-methyl}-biphenyl-4-yl)-propansäure
- 3-[3'-{[Methyl(4-phenoxybenzoyl)amino]-methyl}-2-(3-(trifluormethyl)benzyloxy)-biphenyl-4-yl]-propansäure
- 3-[2-(3-lsopropoxybenzyloxy)-3'-({[3-(4-methoxyphenyl)-propionyl](methyl)amino}-methyl)-biphenyl-4-yl-propansäure
- 3-[2'-(3-Methoxybenzyloxy)-5'-({[3-(3-methoxyphenyl)-propionyl[(methyl)amino}-methyl)-biphenyl-4-yl]-propionsäure
- 3-{2'-Cyclohexylmethoxy-5'-[((hexanoyl)(methyl)amino)-methyl]-biphenyl-4-yl}-propansäure
- 3-{4'-Ethoxy-3'-[((hexanoyl)(methyl)amino)-methyl]-2-propoxybiphenyl-4-yl}-propansäure
- 3-{3'-[((Hexanoyl)(methyl)amino)-methyl]-3,5-dimethoxybiphenyl-4-yl}-propansäure
- 3-[3,5-Diethoxy-3'-({[3-(3-methoxyphenyl)-propionyl](methyl)-amino}-methyl)-biphenyl-4-yl]-propionsäure
- 3-[3'-({[3-(4-Methoxyphenyl)propionyl](methyl)amino}-methyl)-3-propoxybiphenyl-4-yl]-propionsäure
- 3-{3-Cyclopropylmethoxy-3'-[((hexanoyl)(methyl)amino)-methyl]-biphenyl-4-yl}-propansäure
- 3-{3-Ethoxy-4'-fluor-3'-[((hexanoyl)(methyl)amino)-methyl]-biphenyl-4-yl}-propansäure
- 3-[3'-[((Hexanoyl)(methyl)amino)-methyl]-3-(4,4,4-trifluorbutoxy)-biphenyl-4-yl]-propansäure
- 3-{3-Benzyloxy-3'-[((hexanoyl)(methyl)amino)-methyl]-biphenyl-4-yl}-propansäure
- 3-[3'-({[3-(4-Methoxyphenyl)propionyl]methyl)amino}-methyl)-3-(3-(trifluormethyl)benzyloxy)-biphenyl-4-yl]-propionsäure
- 3-[3'-({[3-(3-Methoxyphenyl)propionyl]methyl)amino}-methyl)-3,5-dipropylbiphenyl-4-yl-propionsäure
- 3-{3-(2,2-Dimethylpropyl)-3'-[((hexanoyl)(methyl)amino)-methyl]-biphenyl-4-yl}-propansäure
- 3-{3'-[((Hexanoyl)(methyl)amino)-methyl]-3,5-dimethylbiphenyl-4-yl}-propionsäure
- 3-{3-[((Hexanoyl)(methyl)amino)-methy]-4"-methoxy-1,1';3',1"-terphenyl-4'-yl}-propionsäure
- 3-{3-[((Hexanoyl)(methyl)amino)-methyl]-3"-methoxy-1,1';2',1"-terphenyl-4'-yl}-propionsäure
- 3-[3-({[3-(3-Methoxyphenyl)propionyl](methyl)amino}-methyl)-3"-trifluormethyl-1,1';2',1"-terphenyl-4'-yl]-propionsäure
- 3-{3'-[((Hexanoyl)(methyl)amino)-methyl]-2-[2-(3-isopropoxyphenyl)ethyl]-biphenyl-4-yl}-propionsäure
- 3-{3'-[((Hexanoyl)(methyl)amino)-methyl]-2-[(pyridin-3-ylmethyl)-amino]-biphenyl-4-yl}-propionsäure
- 3-(3'-[((Hexanoyl)(methyl)amino)-methyl]-3-(2-methoxyethylamino)-biphenyl-4-yl]-propionsäure
- Methyl-3-{3,5-diethyl-3'-[((hexanoyl)(methyl)amino)-methyl]-biphenyl-4-yl}-propanoat
- Methyl-3-[3'-{[methyl(3-phenoxybenzoyl}amino]-methyl}-2-(3-(trifluormethyl)benzyloxy)-biphenyl-4-yl-propanoat
- Methyl-3-[3'-{[(3-(biphenyl-4-yl)propionyl)(methyl)amino]-methyl}-2-(3-methoxybenzyloxy)-biphenyl-4-yl]-propanoat
- Ethyl-3-[3'-({[3-(3-methoxyphenyl)propionyl](methyl)amino}-methyl)-2-(4,4,4-trifluorbutoxy)biphenyl-4-yl]-propanoat
- N-[4'-(2-(Hydroxycarbamoyl)-ethyl)-4"-methoxy-1,1';3',1"-terphenyl-3-ylmethyl]-(methyl)-hexanamid.

14. Verbindungen nach Anspruch 1 oder 2, **dadurch gekennzeichnet, dass** sie mindestens eine der folgenden Eigenschaften aufweisen:
- **R1** bedeutet eine Hydroxylgruppe;
- **R2** und **R7** bedeuten eine Alkoxygruppe oder eine Aryloxygruppe, eine Alkylaminogruppe oder eine Polyethergruppe;
- **R3** und **R8** bedeuten ein Wasserstoffatom;
- **R4** bedeutet eine niedere Alkylgruppe mit 1 bis 4 Kohlenstoffatomen;
- **R5** bedeutet eine Alkylgruppe mit 3 bis 8 Kohlenstoffatomen oder eine Arylgruppe;
- **Y** ist ein Sauerstoffatom;
- **V-W** ist eine Gruppierung CH₂-CH₂ oder CH=CH.

15. Verbindungen nach Anspruch 14, **dadurch gekennzeichnet, dass** sie sämtliche nachstehenden Eigenschaften aufweisen:
- **R1** bedeutet eine Hydroxylgruppe;
- **R2** und **R7** bedeuten eine Alkoxygruppe oder eine Aryloxygrupe, eine Alkylaminogruppe oder eine Polyethergruppe;
- **R3** und **R8** bedeuten ein Wasserstoffatom;
- **R4** bedeutet eine niedere Alkylgruppe mit 1 bis 4 Kohlenstoffatomen;
- **R5** bedeutet eine Alkylgruppe mit 3 bis 8 Kohlenstoffatomen oder eine Arylgruppe;
- **Y** ist ein Sauerstoffatom;
- **V-W** bedeutet eine Gruppierung CH₂-CH₂ oder CH=CH.

16. Kosmetische Zusammensetzung, **dadurch gekennzeichnet, dass** sie in einem physiologisch akzeptablen Träger mindestens eine der Verbindungen enthält, die in einem der Ansprüche 1 bis 15 definiert sind.

17. Zusammensetzung nach Anspruch 16, **dadurch gekennzeichnet, dass** die Konzentration der Verbindung(en) nach einem der Ansprüche 1 bis 15 im Bereich von 0,001 bis 3 Gew.-% liegt, bezogen auf das Gesamtgewicht der Zusammensetzung.

18. Kosmetische Verwendung einer Zusammensetzung, wie sie in Anspruch 16 oder 17 definiert ist, zur Körperhygiene oder zur Haarhygiene.

19. Verbindungen nach einem der Ansprüche 1 bis 15 als Arzneimittel.

20. Verwendung einer Verbindung nach einem der Ansprüche 1 bis 15 bei der Herstellung einer Zusammensetzung zur Regulierung und/oder Wiederherstellung des Metabolismus von Hautlipiden.

21. Verwendung einer Verbindung nach einem der Ansprüche 1 bis 15 bei der Herstellung einer Zusammensetzung zur Behandlung von:
- dermatologischen Zuständen, die mit einer Störung der Keratinisierung unter Bezug auf die Zelldifferentiation und Zellproliferation in Verbindung stehen, insbesondere von Acne vulgaris, komedonischer oder polymorpher Akne, Acne rosacea, nodulocystischer Akne, Acne conglobata, seniler Akne oder sekundären Aknen, wie sonnenbedingter Akne, arzneimittelbedingter Akne oder Berufsakne;
- Ichthyosis, ichthyosiformen Zuständen, der Darier'schen Krankheit, palmoplantarem Keratoderma, Leukoplakien und leukoplakiformen Zuständen oder Lichen der Haut oder der Schleimhaut (oral);
- dermatologischen Zuständen mit einer immunallergischen entzündlichen Komponente mit oder ohne Störung der Zellproliferation, insbesondere von Psoriasis der Haut, der Schleimhäute oder der Nägel, von psoriatischem Rheumatismus, Hautatopien, wie Ekzemen, respiratorischer Atopie oder gingivaler Hypertrophie;
- benignen oder malignen dermalen oder epidermalen Proliferationen viralen oder nicht-viralen Ursprungs, insbesondere gemeinen Warzen, Flachwarzen, Epidermodysplasia verruciformis, floriden oder oralen Papillomatosen oder T-Lymphomen;
- Proliferationen, die durch Ultraviolettstrahlung hervorgerufen sein können, insbesondere Basalzell-Epitheliomen und Stachelzell-Epitheliomen;
- präkanzerösen Hautläsionen;
- Immundermatosen, insbesondere Lupus erythematodes,
- bullösen Immunerkrankungen;
- Collagenerkrankungen, insbesondere Skleroderma;
- dermatologischen oder allgemeinen Zuständen mit einer immunologischen Komponente;
- Hauterkrankungen, die durch UV-Strahlungsexposition bedingt sind, lichtinduzierter oder altersbedingter Hautalterung oder von aktinischen Keratosen und Pigmentationen oder anderen Pathologien, die mit altersbedingter oder aktinischer Alterung in Verbindung stehen, insbesondere von Xerose,
- Störungen der Talgdrüsenfunktion, insbesondere von hyperseborrhoischer Akne oder einfacher Seborrhoe;
- Störungen der Narbenbildung oder Schwangerschaftsstreifen;
- Störungen der Pigmentation, wie Hyperpigmentation, Melasma, Hypopigmentation oder Vitiligo;
- Zuständen des Lipidmetabolismus, wie Fettsucht, Hyperlipidämie oder nicht-insulinabhängigem Diabetes;
- entzündlichen Zuständen, wie Arthritis;
- kanzerösen oder präkanzerösen Zuständen;
- Alopezie verschiedenen Ursprungs, insbesondere von durch Chemotherapie oder Bestrahlung bedingter Alopezie;
- Störungen des Immunsystems, wie Asthma, Diabetes mellitus vom Typ I, multipler Sklerose oder anderen selektiven Dysfunktionen des Immunsystems;
- Zuständen des kardiovaskulären Systems, wie Arteriosklerose oder Hypertension.

22. Pharmazeutische Zusammensetzung, **dadurch gekennzeichnet, dass** sie in einem physiologisch akzeptablen Träger mindestens eine der Verbindungen enthält, wie sie in einem der Ansprüche 1 bis 15 definiert sind.

23. Zusammensetzung nach Anspruch 22, **dadurch gekennzeichnet, dass** die Konzentration der Verbindung(en) nach einem der Ansprüche 1 bis 15 im Bereich von 0,001 bis 10 Gew.-% liegt, bezogen auf das Gesamtgewicht der Zusammensetzung.

24. Zusammensetzung nach Anspruch 22, **dadurch gekennzeichnet, dass** die Konzentration der Verbindung(en) nach einem der Ansprüche 1 bis 15 im Bereich von 0,01 bis 1 Gew.-% liegt, bezogen auf das Gesamtgewicht der Zusammensetzung.

## Revendications

1. Composés **caractérisés en ce qu'**ils correspondent à la formule (I) suivante : 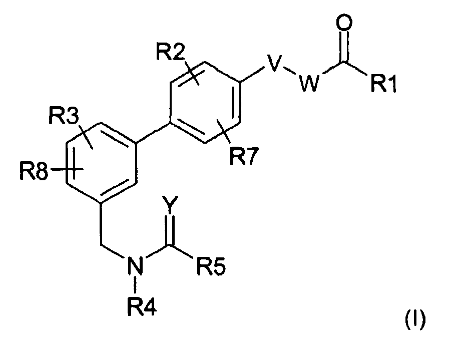 dans laquelle :
- **R1** représente un radical hydroxyle, un radical -OR6 ou un radical hydroxylamine ; R6 étant défini ci-dessous,
- **R2** et **R3,** qui sont identiques ou différents, représentent un atome d'hydrogène, un atome d'halogène, un radical alkyle renfermant de 1 à 12 atomes de carbone, un radical hydroxyle, un radical alcoxy, un radical polyéther, un radical aralkyle, un radical aryle ou un radical amino qui peut être substitué par un ou deux radicaux identiques ou différents choisis parmi un radical alkyle renfermant de 1 à 12 atomes de carbone ou un radical aralkyle ;
- **R4** représente un atome d'hydrogène ou un radical alkyle inférieur renfermant de 1 à 4 atomes de carbone ;
- **R5** représente un radical alkyle renfermant de 1 à 12 atomes de carbone, un radical aryle, un radical aralkyle, un radical hétéroaryle, un radical hétérocyclique ou un radical 9-fluorénylméthyle ;
- **R6** représente un radical alkyle, un radical aryle ou un radical aralkyle ;
- **R7** et **R8**, qui sont identiques ou différents, représentent un atome d'hydrogène, un atome d'halogène, un radical alkyle renfermant de 1 à 12 atomes de carbone, un radical hydroxyle, un radical alcoxy, un radical polyéther, un radical aralkyle, un radical aryle ou un radical amino qui peut être substitué par un ou deux radicaux identiques ou différents choisis parmi un radical alkyle renfermant de 1 à 12 atomes de carbone ou un radical aralkyle ;
- **Y** représente un atome d'oxygène ou un atome de soufre ;
- **V-W** représente une séquence CH₂-CH₂ ou CH = CH,
où :
- « radical alkyle renfermant de 1 à 12 atomes de carbone » signifie une chaîne à base carboné, linéaire, ramifiée ou cyclique, saturée ou insaturée, qui peut être interrompue par un hétéroatome et qui peut être substituée par un ou plusieurs radicaux choisis parmi un atome d'halogène, un radical hydroxyle, un radical alcoxy et un radical hétérocyclique,
- « radical aryle » signifie un radical phényle, un radical biphényle, un radical cinnamyle ou un radical naphtyle qui peut être substitué par un atome d'halogène, un radical CF3, un radical alkyle renfermant de 1 à 12 atomes de carbone, un radical alcoxy, une fonction nitro, un radical polyéther, un radical aryle, un radical benzoyle, un groupe ester alkylique, un groupe acide carboxylique, un radical hydroxyle éventuellement protégé par un groupe acétyle ou un groupe benzoyle ou un radical amino éventuellement protégé par un groupe acétyle ou un groupe benzoyle ou éventuellement substitué par au moins un groupe alkyle renfermant de 1 à 12 atomes de carbone, un radical aralcoxy, un radical phénoxy ou un radical amide H2NCO,
- « radical aralkyle » signifie un radical alkyle renfermant de 1 à 12 atomes de carbone, substitué par un radical aryle ou par un radical hétéroaryle,
- « radical alcoxy » signifie un atome d'oxygène substitué par un radical alkyle renfermant de 1 à 12 atomes de carbone,
- « radical aralcoxy » signifie un atome d'oxygène substitué par un radical aralkyle,
- « radical polyéther » signifie un radical renfermant de 1 à 7 atomes de carbone, interrompu par au moins un atome d'oxygène,
- « radical hétéroaryle » signifie un radical aryle interrompu par un ou plusieurs hétéroatomes, éventuellement substitué par au moins un atome d'halogène, un radical alkyle renfermant de 1 à 12 atomes de carbone, un radical alcoxy, un radical aryle, une fonction nitro, un radical polyéther, un radical hétéroaryle, un radical benzoyle, un groupe ester alkylique, un groupe acide carboxylique, un groupe hydroxyle éventuellement protégé par un groupe acétyle ou un groupe benzoyle ou un radical amino éventuellement protégé par un groupe acétyle ou un groupe benzoyle ou éventuellement substitué par au moins un groupe alkyle renfermant de 1 à 12 atomes de carbone,
et les sels et composés de formule (I).

2. Composés selon la revendication 1, **caractérisés en ce qu'**ils sont procurés sous la forme de sels d'un métal alcalin ou d'un métal alcalino-terreux ou de sels d'une amine organique.

3. Composés selon la revendication 1, **caractérisés en ce qu'**ils sont procurés, lorsqu'ils renferment un groupe fonctionnel amine, sous la forme de sels d'un acide inorganique ou de sels d'un acide organique.

4. Composés selon l'une des revendications 1 à 3, **caractérisés en ce que** les radicaux alkyle renfermant de 1 à 12 atomes de carbone sont choisis parmi le radical méthyle, le radical éthyle, le radical propyle, le radical isopropyle, le radical butyle, le radical tert-butyle, le radical iso-amyle, le radical amyle, le radical hexyle, le radical heptyle, le radical octyle, le radical décyle, le radical cyclohexyle, le radical méthylcyclohexyle, le radical méthylcyclobutyle, le radical méthylcyclopentyle ou le radical méthylcyclohexyle.

5. Composés selon l'une quelconque des revendications 1 à 4, **caractérisés en ce que** les radicaux alkyle inférieurs renfermant de 1 à 4 atomes de carbone sont choisis parmi le radical méthyle, le radical éthyle, le radical propyle, le radical méthylcyclopropyle, le radical isopropyle, le radical tert-butyle ou le radical n-butyle.

6. Composés selon l'une quelconque des revendications 1 à 5, **caractérisés en ce que** les radicaux aryle sont choisis parmi le radical phényle, le radical biphényle, le radical cinnamyle ou le radical naphtyle, qui peut être substitué par un atome d'halogène, un radical CF₃, un radical alkyle renfermant de 1 à 12 atomes de carbone, un radical alcoxy, un groupe fonctionnel nitro, un radical polyéther, un radical aryle, un radical benzoyle, un groupe ester alkylique, un groupe acide carboxylique, un radical hydroxyle éventuellement protégé par un groupe acétyle ou un groupe benzoyle ou un radical amino éventuellement protégé par un groupe acétyle ou un groupe benzoyle ou éventuellement substitué par au moins un groupe alkyle renfermant de 1 à 12 atomes de carbone, un radical aralcoxy, un radical phénoxy ou un radical amide H₂NCO.

7. Composés selon l'une quelconque des revendications 1 à 6, **caractérisés en ce que** les radicaux aralkyle sont des radicaux alkyle renfermant de 1 à 12 atomes de carbone, qui sont substitués par un radical aryle ou par un radical hétéroaryle.

8. Composés selon l'une quelconque des revendications 1 à 7, **caractérisés en ce que** l'atome d'halogène est choisi parmi un atome de fluor, un atome de chlore, un atome de brome ou un atome d'iode.

9. Composés selon l'une quelconque des revendications 1 à 8, **caractérisés en ce que** les radicaux alcoxy sont choisis parmi le radical méthoxy, le radical éthoxy, le radical isopropyloxy, le radical n-propyloxy, le radical tert-butoxy, le radical n-butoxy, le radical n-pentyloxy, le radical n-hexyloxy, le radical cyclopropylméthoxy, le radical cyclobutylméthoxy, le radical cyclopentylméthoxy ou le radical cyclohexylméthoxy.

10. Composés selon l'une quelconque des revendications 1 à 9, **caractérisés en ce que** les radicaux polyéther sont choisis parmi des radicaux renfermant de 1 à 7 atomes de carbone, qui sont interrompus par au moins un atome d'oxygène et de préférence les radicaux tels que le radical méthoxyéthoxy, le radical éthoxyéthoxy ou le radical méthoxyéthoxyéthoxy.

11. Composés selon l'une quelconque des revendications 1 à 10, **caractérisés en ce que** le radical hétéroaryle est choisi parmi le groupe constitué d'un radical pyridyle, d'un radical furyle, d'un radical thiényle, d'un radical isoxazolyle, d'un radical oxadiazolyle, d'un radical oxazolyle, d'un radical isothiazolyle, d'un radical quinozalinyle, d'un radical benzothiadiazolyle, d'un radical benzimidazolyle, d'un radical indolyle et d'un radical benzofuranyle, qui sont éventuellement substitués par au moins un atome d'halogène, un radical alkyle renfermant de 1 à 12 atomes de carbone, un radical alcoxy, un radical aryle, un groupe fonctionnel nitro, un radical polyéther, un radical hétéroaryle, un radical benzoyle, un groupe ester alkylique, un groupe acide carboxylique, un groupe hydroxyle éventuellement protégé par un groupe acétyle ou un groupe benzoyle ou un radical amino éventuellement protégé par un groupe acétyle ou un groupe benzoyle ou éventuellement substitué par au moins un groupe alkyle renfermant de 1 à 12 atomes de carbone.

12. Composés selon l'une quelconque des revendications 1 à 11, **caractérisés en ce que** le radical hétérocyclique est choisi parmi un radical morpholino, un radical pyrrolidino, un radical pipéridino, un radical pipérazino, un radical 2-oxopipéridin-1-yle et un radical 2-oxopyrrolidin-1-yle qui sont éventuellement substitués par au moins un radical alkyle renfermant de 1 à 12 atomes de carbone, un radical alcoxy, un radical aryle, un groupe fonctionnel nitro, un radical polyéther, un radical hétéroaryle, un radical benzoyle, un groupe ester alkylique, un groupe acide carboxylique, un groupe hydroxyle éventuellement protégé par un groupe acétyle ou un groupe benzoyle ou un radical amino éventuellement protégé par un groupe acétyle ou un groupe benzoyle ou éventuellement substitué par au moins un groupe alkyle renfermant de 1 à 12 atomes de carbone.

13. Composés selon la revendication 1, **caractérisés en ce qu'**ils sont pris, seuls ou sous la forme de mélanges, à partir du groupe constitué de :
- l'acide 3-{3'-[((méthyl)(octanoyl)amino)méthyl]biphényl-4-yl}acrylique
- l'acide 3-{3'-[((méthyl)(octanoyl)amino)méthyl]biphényl-4-yl}propanoïque
- l'acide 3-{3-fluoro-3'-[((méthyl)(octanoyl)amino)méthyl]biphényl-4-yl}acrylique
- l'acide 3-{3-fluoro-3'-[((méthyl)(octanoyl)amino)méthyl]biphényl-4-yl}propanoïque
- l'acide 3-[3'-((octanoylamino)méthyl)biphényl-4-yl]acrylique
- l'acide 3-[3'-((octanoylamino)méthyl)biphényl-4-yl}propanoïque
- l'acide 3-{3-hydroxy-3'-[((méthyl)(octanoyl)amino)méthyl]biphényl-4-yl}propanoïque
- l'acide 3-{2-butoxy-3'-[((méthyl)(octanoyl)amino)méthyl]biphényl-4-yl}propanoïque
- l'acide 3-{2-butoxy-3'-[((hexanoyl)(méthyl)amino)méthyl]biphényl-4-yl}propanoïque
- l'acide 3-{2-butoxy-3'-[((méthyl)(pentanoyl)amino)méthyl]biphényl-4-yl}propanoïque
- l'acide 3-{2-butoxy-3'-[((heptanoyl)(méthyl)amino)méthyl]biphényl-4-yl}propanoïque
- l'acide 3-(2-butoxy-3'-{[(4-éthoxybenzoyl)(méthyl)amino]méthyl}biphényl-4-yl)propanoïque
- l'acide 3-(2-butoxy-3'-{[(4-butoxybenzoyl)(méthyl)amino]méthyl}biphényl-4-yl)propanoïque
- l'acide 3-{3'-[((benzoyl)(méthyl)amino)méthyl]-2-butoxy-biphényl-4-yl}propanoïque
- l'acide 3-(2-butoxy-3'-{[(4-méthoxybenzoyl)(méthyl)amino]méthyl}biphényl-4-yl)propanoïque
- l'acide 3-(2-butoxy-3'-{[(3-méthoxybenzoyl)(méthyl)amino]méthyl}biphényl-4-yl)propanoïque
- l'acide (E)-3-{2-fluoro-3'-[((méthyl)(octanoyl)amino)méthyl]biphényl-4-yl}acrylique
- l'acide 3-{2-fluoro-3'-[((méthyl)(octanoyl)amino)méthyl]biphényl-4-yl}propanoïque
- l'acide 3-{2-(2-méthoxyéthoxy)-3'-[((méthyl)(octanoyl)amino)méthyl]biphényl-4-yl}propanoïque
- l'acide 3-{2-(3-méthylbutoxy)-3'-[((méthyl)(octanoyl)amino)méthyl]biphényl-4-yl}propanoïque
- l'acide 3-{2-(3-chloropropoxy)-3'-[((méthyl)(octanoyl)amino)méthyl]biphényl-4-yl}propanoïque
- l'acide 3-{2-méthoxy-3'-[((méthyl)(octanoyl)amino)méthyl]biphényl-4-yl}propanoïque
- l'acide 3-{2-méthyl-3'-[((méthyl)(octanoyl)amino)méthyl]biphényl-4-yl}propanoïque
- l'acide 3-(3'-{[méthyl(4-phénylbutyryl)amino]méthyl}biphényl-4-yl)propanoïque
- l'acide (E)-3-{2'-méthyl-5'-[((méthyl)(octanoyl)amino)méthyl]biphényl-4-yl}acrylique
- l'acide 3-{2'-méthyl-5'-[((méthyl)(octanoyl)amino)méthyl]biphényl-4-yl}propanoïque
- l'acide (E)-3-{2-benzyloxy-3'-[((méthyl)(octanoyl)amino)méthyl]biphényl-4-yl}acrylique
- l'acide 3-{2-benzyloxy-3'-[((méthyl)(octanoyl)amino)méthyl]biphényl-4-yl}propanoïque
- l'acide 3-(3'-[((méthyl)(octanoyl)amino)méthyl]-2-propoxybiphényl-4-yl}propanoïque
- l'acide (E)-3-{3,5-difluoro-3'-[((méthyl)(octanoyl)amino)méthyl]biphényl-4-yl}acrylique
- l'acide 3-{3,5-difluoro-3'-[((méthyl)(octanoyl)amino)méthyl]biphényl-4-yl}propanoïque
- l'acide 3-(3'-{[(4-butoxybenzoyl)(méthyl)amino]méthyl}biphényl-4-yl)propanoïque
- l'acide 3-(3'-{[(4-butoxybenzoyl)(éthyl)amino]méthyl}biphényl-4-yl)propanoïque
- l'acide 3-{2-cyclopropylméthoxy-3'-[((méthyl)(octanoyl)amino)méthyl]biphényl-4-yl}propanoïque
- l'acide 3-{2-éthoxy-3'-[((méthyl)(octanoyl)amino)méthyl]biphényl-4-yl}propanoïque
- l'acide 3-[3'-[((méthyl)(octanoyl)amino)méthyl]-2-(3,3,3-trifluoropropoxy)biphényl-4-yl]propanoïque
- l'acide 3-[3'-[((méthyl)(octanoyl)amino)méthyl]-2-(4,4,4-trifluorobutoxy)biphényl-4-yl]propanoïque
- l'acide 3-{2-(3-hydroxypropoxy)-3'-[((méthyl)(octanoyl)amino)méthyl]biphényl-4-yl}propanoïque
- l'acide 3-{2-(4-hydroxybutoxy)-3'-[((méthyl)(octanoyl)amino)méthyl]biphényl-4-yl}propanoïque
- l'acide 3-{2-(3-fluorobenzyloxy)-3'-[((méthyl)(octanoyl)amino)méthyl]biphényl-4-yl}propanoïque
- l'acide 3-{2-(4-fluorobenzyloxy)-3'-[((méthyl)(octanoyl)amino)méthyl]biphényl-4-yl}propanoïque
- l'acide 3-{3'-[((méthyl)(octanoyl)amino)méthyl]-2-(pentyloxy)biphényl-4-yl}propanoïque
- l'acide 3-{3'-[((hexanoyl)(méthyl)amino)méthyl]-2-(pentyloxy)biphényl-4-yl}propanoïque
- l'acide 3-{2-(2-éthoxyéthoxy)-3'-[((hexanoyl)(méthyl)amino)méthyl]biphényl-4-yl}propanoïque
- l'acide 3-{2-(2-(diéthylamino)éthoxy)-3'-[((méthyl)(octanoyl)amino)méthyl]biphényl-4-yl}propanoïque
- l'acide 3-[3'-[((méthyl)(octanoyl)amino)méthyl]-2-(2-(morpholin-4-yl)éthoxy)biphényl-4-yl]propanoïque
- l'acide 3-{2-amino-3'-[((hexanoyl)(méthyl)amino)méthyl]biphényl-4-yl}propanoïque
- l'acide 3-{2-butylamino-3'-[((hexanoyl)(méthyl)amino)méthyl]biphényl-4-yl}propanoïque
- l'acide 3-{2-benzylamino-3'-[((hexanoyl)(méthyl)amino)méthyl]biphényl-4-yl}propanoïque
- l'acide 3-{2-diéthylamino-3'-[((hexanoyl)(méthyl)amino)méthyl]biphényl-4-yl}propanoïque
- l'acide 3-{3'-[((hexanoyl)(méthyl)amino)méthyl]-2-(propylamino)biphényl-4-yl}propanoïque
- l'acide 3-{2-(4-fluorobenzylamino)-3'-[((hexanoyl)(méthyl)amino)méthyl]biphényl-4-yl}propanoïque
- l'acide 3-{2-butylamino-3'-[((méthyl)(octanoyl)amino)méthyl]biphényl-4-yl}propanoïque
- l'acide 3-{2-benzylamino-3'-[((méthyl)(octanoyl)amino)méthyl]biphényl-4-yl}propanoïque
- l'acide 3-{2-diéthylamino-3'-[((méthyl)(octanoyl)amino)méthyl]biphényl-4-yl}propanoïque
- l'acide 3-{3'-[((méthyl)(octanoyl)amino)méthyl]-2-(propylamino)biphényl-4-yl}propanoïque
- l'acide 3-{2-(4-fluorobenzylamino)-3'-[((méthyl)(octanoyl)amino)méthyl]biphényl-4-yl}propanoïque
- l'acide 3-{2-cyclohexylméthoxy-3'-[((méthyl)(octanoyl)amino)méthyl]biphényl-4-yl}propanoïque
- l'acide 3-{2-cyclopentylméthoxy-3'-[((méthyl)(octanoyl)amino)méthyl]biphényl-4-yl}propanoïque
- du N-[2'-(2-cyclobutyléthoxy)-4'-(2-(hydroxycarbamoyl)éthyl)biphényl-3-ylméthyl](méthyl)octanamide
- l'acide 3-[3'-[((méthyl)(octanoyl)amino)méthyl]-2-(3-(trifluorométhyl)benzyloxy)biphényl-4-yl]propanoïque
- l'acide 3-[3'-[((hexanoyl)(méthyl)amino)méthyl]-2-(4-(trifluorométhyl)benzyloxy)biphényl-4-yl]propanoïque
- l'acide 3-{2-(3-carbamoylbenzyloxy)-3'-[((méthyl)(octanoyl)amino)méthyl]biphényl-4-yl}propanoïque
- l'acide 3-[3'-[((méthyl)(octanoyl)amino)méthyl]-2-(2-(pipérazin-1-yl)éthoxy)biphényl-4-yl]propanoïque
- l'acide 3-[3'-[((méthyl)(octanoyl)amino)méthyl]-2-(2-(pyrrolidin-1-yl)éthoxy)biphényl-4-yl]propanoïque
- l'acide 3-[2-(3-méthoxybenzyloxy)-3'-({[3-(3-méthoxyphényl)propionyl](méthyl)amino}méthyl)bi phényl-4-yl]propanoïque
- l'acide 3-[2-(4-(tert-butyl)benzyloxy)-3'-({méthyl[3-(3-phénoxyphényl)propionyl]amino}méthyl)biphényl-4-yl]propanoïque
- l'acide 3-(2-(3,5-diméthoxybenzyloxy)-3'-{[méthyl(3-phénoxybenzoyl)amino]méthyl}biphényl-4-yl)propanoïque
- l'acide 3-[3'-{[méthyl(4-phénoxybenzoyl)amino]méthyl}-2-(3-(trifluorométhyl)benzyloxy)biphényl-4-yl]propanoïque
- l'acide 3-[2-(3-isopropoxybenzyloxy)-3'-({[3-(4-méthoxyphényl)propionyl](méthyl)amino}méthyl) biphényl-4-yl]propanoïque
- l'acide 3-[2'-(3-méthoxybenzyloxy)-5'-({[3-(3-méthoxyphényl)propionyl](méthyl)amino}méthyl) biphényl-4-yl]propanoïque
- l'acide 3-{2'-cyclohexylméthoxy-5'-[((hexanoyl)(méthyl)amino)méthyl]biphényl-4-yl}propanoïque
- l'acide 3-{4'-éthoxy-3'-[((hexanoyl)(méthyl)amino)méthyl]-2-propoxybiphényl-4-yl}propanoïque
- l'acide 3-{3'-[((hexanoyl)(méthyl)amino)méthyl]-3,5-diméthoxybiphényl-4-yl}propanoïque
- l'acide 3-[3,5-diéthoxy-3'-({[3-(3-méthoxyphényl)propionyl](méthyl)amino}méthyl) biphényl-4-yl]propanoïque
- l'acide 3-[3'-({[3-(4-méthoxyphényl)propionyl](méthyl)amino}méthyl)-3-propoxybiphényl-4-yl]propanoïque
- l'acide 3-{3-cyclopropylméthoxy-3'-[((hexanoyl)(méthyl)amino)méthyl]biphényl-4-yl}propanoïque
- l'acide 3-{3-éthoxy-4'-fluoro-3'-[((hexanoyl)(méthyl)amino)méthyl]biphényl-4-yl}propanoïque
- l'acide 3-[3'-[((hexanoyl)(méthyl)amino)méthyl]-3-(4,4,4-trifluorobutoxy)biphényl-4-yl]propanoïque
- l'acide 3-{3-benzyloxy-3'-[((hexanoyl)(méthyl)amino)méthyl]biphényl-4-yl}propanoïque
- l'acide 3-[3'-({[3-(4-méthoxyphényl)propionyl](méthyl)amino}méthyl)-3-(3-(trifluorométhyl)benzyloxy)biphényl-4-yl]propanoïque
- l'acide 3-[3'-({[3-(3-méthoxyphényl)propionyl](méthyl)amino}méthyl)-3,5-dipropylbiphényl-4-yl]propanoïque
- l'acide 3-{3-(2,2-diméthylpropyl)-3'-[((hexanoyl)(méthyl)amino)méthyl]biphényl-4-yl}propanoïque
- l'acide 3-{3'-[((hexanoyl)(méthyl)amino)méthyl]-3,5-diméthylbiphényl-4-yl}propanoïque
- l'acide 3-{3-[((hexanoyl)(méthyl)amino)méthyl]-4"-méthoxy-1,1';3',1"-terphényl-4'-yl}propanoïque
- l'acide 3-{3-[((hexanoyl)(méthyl)amino)méthyl]-3''-méthoxy-1,1';2',1''-terphényl-4'-yl}propanoïque
- l'acide 3-[3-({[3-(3-méthoxyphényl)propionyl](méthyl)amino}méthyl)-3''-trifluorométhyl-1,1';2',1''-terphényl-4'-yl]propanoïque
- l'acide 3-{3'-[((hexanoyl)(méthyl)amino)méthyl]-2-[2-(3-isopropoxyphényl)éthyl]biphényl-4-yl}propanoïque
- l'acide 3-{3'-[((hexanoyl)(méthyl)amino)méthyl]-2-[(pyridin-3-ylméthyl)amino]biphényl-4-yl}propanoïque
- l'acide 3-[3'-[((hexanoyl)(méthyl)amino)méthyl]-3-(2-méthoxyéthylamino)biphényl-4-yl]propanoïque
- du 3-{3,5-diéthyl-3'-[((hexanoyl)(méthyl)amino)méthyl]biphényl-4-yl}propanoate de méthyle
- du 3-[3'-{[méthyl(3-phénoxybenzoyl)amino]méthyl}-2-(3-(trifluorométhyl)benzyloxy)biphényl-4-yl]propanoate de méthyle
- du 3-[3'-{[(3-(biphényl-4-yl)propionyl)(méthyl)amino]méthyl}-2-(3-méthoxybenzyloxy)biphényl-4-yl]propanoate de méthyle
- du 3-[3'-({[3-(3-méthoxyphényl)propionyl](méthyl)amino}méthyl)-2-(4,4,4-trifluorobutoxy)biphényl-4-yl]propanoate d'éthyle
- du N-[4'-(2-(hydroxycarbamoyl)éthyl)-4''-méthoxy-1,1';3',1''-terphényl-3-ylméthyl](méthyl)hexanamide.

14. Composés selon la revendication 1 ou 2, **caractérisés en ce qu'**ils présentent au moins l'une des caractéristiques suivantes :
- **R1** représente un radical hydroxyle ;
- **R2** et **R7** représentent un radical alcoxy ou un radical aryloxy, un radical alkylamino ou un radical polyéther ;
- **R3** et **R8** représentent un atome d'hydrogène ;
- **R4** représente un radical alkyle inférieur renfermant de 1 à 4 atomes de carbone ;
- **R5** représente un radical alkyle renfermant de 3 à 8 atomes de carbone ou un radical aryle ;
- **Y** est un atome d'oxygène ;
- la liaison **V-W** est une séquence CH2-CH2 ou CH=CH.

15. Composés selon la revendication 14, **caractérisés en ce qu'**ils présentent au moins l'une des caractéristiques suivantes :
- **R1** représente un radical hydroxyle ;
- **R2** et **R7** représentent un radical alcoxy ou un radical aryloxy, un radical alkylamino ou un radical polyéther ;
- **R3** et **R8** représentent un atome d'hydrogène ;
- **R4** représente un radical alkyle inférieur renfermant de 1 à 4 atomes de carbone ;
- **R5** représente un radical alkyle renfermant de 3 à 8 atomes de carbone ou un radical aryle ;
- **Y** est un atome d'oxygène ;
- la liaison **V-W** est une séquence CH2-CH2 ou CH=CH.

16. Composition cosmétique, **caractérisée en ce qu'**elle comprend, dans un véhicule physiologiquement acceptable, au moins l'un des composés tels que définis dans l'une quelconque des revendications 1 à 15.

17. Composition selon la revendication 16, **caractérisée en ce que** la concentration de composé(s) selon l'une des revendications 1 à 15 est comprise entre 0,001 % et 3 % en poids, par rapport au poids total de la composition.

18. Utilisation cosmétique d'une composition telle que définie dans l'une ou l'autre des revendications 16 et 17 pour l'hygiène du corps ou des cheveux.

19. Composés selon l'une quelconque des revendications 1 à 15, en tant que médicament.

20. Utilisation d'un composé selon l'une quelconque des revendications 1 à 15 pour la fabrication d'une composition destinée à réguler et/ou à rétablir le métabolisme de lipides de la peau.

21. Utilisation d'un composé selon l'une quelconque des revendications 1 à 15 pour la fabrication d'une composition destinée au traitement :
- de pathologies dermatologiques liées à un trouble de kératinisation impliquant une différenciation et une prolifération, en particulier l'acné vulgaire, l'acné comedonica ou polymorphe, l'acné rosacée, l'acné nodulokystique, l'acné conglobata, l'acné sénile ou des acnés secondaires, telles que l'acné solaire, médicamenteuse ou professionnelle,
- de l'ichthyose, de pathologies ichthyosiformes, de la maladie de Darier, de la kératodermie palmo-plantaire, de la leucoplasie et de pathologies leucoplasiformes, ou du lichen cutané ou muqueux (oral),
- de pathologies dermatologiques avec une composante immuno-allergique inflammatoire, avec ou sans trouble de la prolifération cellulaire, en particulier le psoriasis cutané, muqueux ou unguéal, le rhumatisme psoriatique, l'atopie cutanée, telle que l'eczéma, l'atopie respiratoire ou l'hypertrophie gingivale,
- de proliférations dermiques ou épidermiques, bénignes ou malignes, d'origine virale ou non virale, en particulier des verrues vulgaires, des verrues planes, l'épidermodysplasie verruciforme, des papillomatoses développées ou orales, ou le lymphome T,
- de proliférations qui peuvent être induites par le rayonnement ultraviolet, en particulier l'épithéliome à cellules basales ou à cellules malpighiennes,
- de lésions cutanées pré-cancéreuses, en particulier des kérato-acanthomes,
- de dermatoses immunes, en particulier le lupus érythémateux,
- de maladies bulleuses immunes,
- de maladies du collagène, en particulier le scléroderme,
- de pathologies dermatologiques ou générales avec une composante immunologique,
- de troubles cutanés dus à une exposition à un rayonnement UV, d'un vieillissement cutané photo-induit ou chronologique ou de kératoses et de pigmentations actiniques, ou toute pathologie associée à un vieillissement chronologique ou actinique, en particulier la xérose,
- de troubles de la fonction sébacée, en particulier l'hyperséborrhée de l'acné ou la séborrhée simple,
- de troubles de cicatrisation et de vergetures,
- de troubles de pigmentation, tels que l'hyperpigmentation, le mélasme, l'hypopigmentation ou le vitiligo,
- de pathologies du métabolisme des lipides, telles que l'obésité, l'hyperlipidémie ou le diabète non insulino-dépendant,
- de pathologies inflammatoires, telles que l'arthrite,
- de pathologies cancéreuses ou pré-cancéreuses,
- de l'alopécie de diverses origines, en particulier l'alopécie dû à la chimiothérapie ou un rayonnement,
- de troubles du système immunitaire, tels que l'asthme, le diabète sucré du type I, la sclérose en plaques ou d'autres dysfonctionnements sélectifs du système immunitaire,
- de pathologies du système cardiovasculaire, telles que l'artériosclérose ou l'hypertension.

22. Composition pharmaceutique, **caractérisée en ce qu'**elle comprend, dans un véhicule physiologiquement acceptable, au moins l'un des composés tels que définis dans l'une quelconque des revendications 1 à 15.

23. Composition selon la revendication 22, **caractérisée en ce que** la concentration du composé(s) selon l'une des revendications 1 à 15 est comprise entre 0,001 % et 10 % en poids, par rapport au poids total de la composition.

24. Composition selon la revendication 22, **caractérisée en ce que** la concentration du composé(s) selon l'une des revendications 1 à 15 est comprise entre 0,01 % et 1 % en poids, par rapport au poids total de la composition.
